# EUROPEAN PATENT SPECIFICATION

(11) **EP 0 909 249 B1**
(45) Date of publication and mention of the grant of the patent: **27.04.2005**
(21) Application number: 97938244.7
(22) Date of filing: 08.08.1997
(51) Int. Cl.: A61M 25/00, A61M 25/01, B65D 83/10

(54) **PRELUBRICATED URINARY CATHETER AND PACKAGE ASSEMBLY**
GESCHMIERTER HARNKATHETER UND VERPACKUNGSSYSTEM
CATHETER URINAIRE PRELUBRIFIE ET ENSEMBLE D'EMBALLAGE

(30) Priority: 14.08.1996 US 689796
(43) Date of publication of application: 21.04.1999
(60) Divisional application: 04022127.7
(73) Proprietor: Medical Technologies of Georgia, Inc., Alpharetta, GA 30022 (US)
(72) Inventor: GOLDEN, John, H., Conyers, GA 30208 (US); STARKE, Richard, N., Alpharetta, GA 30202 (US); ROBLING, Brent, D., Raleigh, NC 27613 (US); POWELL, Billy, J., Adairsville, GA 30103 (US)
(74) Representative: Ouzman, Beverley Nicola Claire
(86) International application number: PCT/US1997/014130
(87) International publication number: WO 1998/006642

(56) References cited:
- EP-A- 0 677 299
- US-A- 3 035 691
- US-A- 3 648 704
- US-A- 3 934 721
- US-A- 3 967 728
- US-A- 5 226 530
- US-A- 5 454 798
- US-A- 5 472 428

## Description

### BACKGROUND OF THE INVENTION

This invention relates generally to urinary catheters and more prelubricated intermittent urinary catheter and package assemblies.

Heretofore, single use catheters designed for intermittent or self catheterization have been supplied in various packaging configurations. Examples of such packaging arrangements are illustrated in the following patents:

| U.S. Pat. No. | Inventor | Issue Date | Class/Subclass |
|---|---|---|---|
| 2,329,360 | Salfisberg | 9/1943 | 383/200 |
| 2,552,870 | Scherer | 5/1951 | 383/210 |
| 2,856,932 | Griffitts | 10/1958 | 206/364 |
| 3,035,691 | Rasmussen et al. | 5/1962 | 206/364 |
| 3,642,126 | Kurtz et al. | 2/1972 | 383/200 |
| 3,648,704 | Jackson | 3/1972 | 206/364 |
| 3,910,410 | Shaw | 10/1975 | 206/363 |
| 3,934,721 | Juster et al. | 1/1976 | 206/364 |
| 4,140,127 | Cianci et al. | 2/1979 | 206/364 |
| 5,226,530 | Golden | 7/1993 | 206/364 |

To be used, the catheter was removed from the package and then used. One of the problems with such a packaging arrangement is that the catheter can easily become contaminated during removal from the package thereby transferring that contamination to the urethral tract as the catheter is used. Another problem is that the catheter itself must be handled as it is inserted in the urethral tract. This also contributed to the likelihood of contamination of the catheter to be transferred to the urethral tract.

Other arrangements supplied a catheter kit with the catheter inside a urine collection bag. The catheter kit was then housed in a separate sterile package. Examples of such catheter kits are illustrated in the following patents:

| U.S. Pat. No. | Inventor | Issue Date | Class/Subclass |
|---|---|---|---|
| 4,204,527 | Wu et al. | 5/1980 | 206/364 |
| 4,230,115 | Walz, Jr. et al. | 10/1980 | 206/364 |
| 4,246,909 | Wu et al. | 1/1981 | |
| 4,652,259 | O'Neil | 3/1987 | . 604/54 |
| 5.147.341 | Starke et al. | 9/1992 | 604/349 |

These arrangements facilitated the manipulation of the catheter through the collection bag to insert the catheter into the urethral tract but required separate packaging to insure sterility of the catheter prior to use.

Another problem with the prior art, especially where the catheter is inserted from a bag through which the catheter must be gripped for manipulation, is that the procedure requires relatively good dexterity on the part of the user to carry out the manipulation. Unfortunately, many users of intermittent catheterization do not have very good dexterity thus effectively preventing self catheterization by these users.

Another problem associated with the prior art is that it is difficult to insure that the catheter is properly lubricated for insertion. This is because the lubricant is designed to liquify at body temperature and frequently becomes sufficiently liquid during storage to coat the inside of the package carrying the catheter and also the complete catheter. As a result, the prior art has frequently supplied the lubricant in a separate container. With most prior art prelubricated catheters, the catheter is stowed in a curved shape. Sometimes, the catheter retained this curved shape during use thereby making the catheter more difficult and uncomfortable to use. A prelubricated urinary catheter according to the preamble of claim 1 is known for example from US-A-5 454 798.

### SUMMARY OF THE INVENTION

These and other problems and disadvantages associated with the prior art are overcome by the invention disclosed herein by providing a urinary catheter and package assembly which provides a prelubricated catheter in a straight condition for intermittent use. This serves to facilitate the use of the catheter. The catheter is used without having to be completely removed from the package to reduce the likelihood of contamination and the package can be used as a drainage extension to further facilitate the use of the catheter. Guide means is provided to locate the catheter as it is extended from the package to facilitate gripping the catheter and controlling its position relative to the package. Extension control means is provided to help the user manipulate the catheter within the package as it is being extended. The extension control means is designed to grip the catheter in such a way that the user does not have to maintain their grip on the catheter as the package is being moved relative to the catheter during manipulation for extension. The catheter is inserted using the packaging so that the user never has to touch the catheter itself. The packaging is such that the lubricant is applied to the projecting end of the catheter in the package in such a way as to insure that the catheter is adequately lubricated for use. Manually operated lubricate applicator means may be provided to insure that lubricant is forced onto the catheter within the package either before or during extension. Because the drainage end of the catheter can be left within one end of the packaging, the rest of packaging can be pulled over the drainage end of the catheter to act as a drainage extension for the catheter since the remaining lubricant within the packaging serves as a sufficient seal between the catheter and the packaging to prevent leakage.

The apparatus of the invention is directed to a prelubricated urinary catheter and package assembly for use in draining the bladder through the urethra as defined in claim 1 comprising a package with opposed first and second ends and defining a sealed elongate catheter receiving chamber therein extending for a minimum prescribed length along the length of the package; an elongate catheter with opposed projecting and drainage ends and a maximum length less than the minimum prescribed length of the catheter receiving chamber in the package, the catheter being carried in the sealed elongate catheter receiving chamber in a generally straight condition with the projecting end of the catheter located at that end of the catheter receiving chamber with the opening initiating means so that the projecting end of the catheter can pass out of the catheter receiving chamber when a manual opening is formed in the end of the package; and guide means operatively associated with the first end of the package and defining a catheter guide passage oriented generally along the longitudinal axis through the package where the catheter receiving opening communicates with the elongate catheter receiving chamber and is operatively associated with the first opening initiating means so that the catheter receiving opening remains sealed until the package is manually opened whereupon said projecting end of the catheter slidably passes out of the catheter receiving chamber through the catheter receiving opening to guide the passage of the catheter out of the catheter receiving chamber.

One or both of the ends of the package may include opening initiating means for controlling the manual opening of the catheter chamber at that end associated with the opening initiating means. The opening initiating means may include a tear initiating arrangement, a peel apart arrangement, or an axial cut in the end of the package. The opening initiating means may be constructed and arranged so as to not expose the catheter to a contaminated surface as it is extended from the package.

The guide means may comprise a preformed guide member defining the catheter guide passage therethrough held in place within the sealed catheter receiving chamber adjacent the first end of the package. The guide member may be flexible or rigid. The guide means may alternatively comprise a guide strip bonded to one side of the package at the first end so that the guide strip lies adjacent the sealed catheter receiving chamber in the package before the package is opened and extends across the opening in the first end of the package when the package is opened, where the guide passage is formed by the guide strip defining a guide recess thereacross generally in alignment with the first axis of the package and forming an opening thereinto along the length thereof facing the opposite side of the package and by the opposite of the package extending across the opening into the recess to close same. The guide strip may be affixed to the exterior or interior of the side of the package. Where the guide strip is affixed to the exterior of the package, that side of the package immediately adjacent guide strip is affixed to and extends into the guide recess in the guide strip to form a conforming recess therein matching the guide recess in the guide strip so that the catheter receiving opening is defined between the sides of the package.

Gripping means may be provided for the user to grip the catheter at the guide means so as to longitudinally fix the catheter relative to the guide means. The gripping means may be incorporated in the guide means to facilitate manufacture and assembly of the package and catheter assembly. Alternatively, the catheter can be gripped by the user pressing that side of the package opposite the guide strip into the recess in the guide strip to frictionally engage the catheter between the guide strip and the opposite side of the package.

The extension control means may comprise gripping members sized and configured so as to grip the catheter tube of the catheter sufficiently to require a minimum threshold force to move the catheter with respect to the extension control means. The minimum threshold force may be selected at a value such that the package can be moved relatively to the catheter when manually released yet the catheter can be gripped through the package to move the catheter through the extension control means. The extension control means may also be arranged so as to act as a one-way clutch mechanism to easily permit extension of the catheter from the package but to substantially prevent retraction of the catheter back into the package. The extension control means may be incorporated in the guide means as control protrusions or may be a separate control member that cooperates with the guide means to engage the catheter.

The lubricating means may include a lubricant reservoir formed in the package which carries a prescribed quantity of lubricant for application to the catheter during use. The catheter may also be lubricated with additional lubricant in the catheter receiving chamber and the catheter receiving passage of the guide means. The lubricating means may be constructed and arranged such that the user can manually press in on the package in the vicinity of the lubricant reservoir to force the lubricant onto the catheter as it passes the lubricant reservoir during extension of the catheter from the package. The guide means may also be constructed and arranged to permit the lubricant to be injected into the guide passage around the catheter from the lubricant reservoir.

The package may be comprised of a base sheet of material in which a catheter receiving recess is formed with the recess opening onto one side of the sheet to form a loading opening extending along the length of the recess and with the catheter receiving recess having a length and cross-sectional size and shape corresponding to that of the catheter receiving chamber; and, a cover sheet of material covering the loading opening with the base sheet and the cover sheet sealed to each other around the loading opening to form the sealed catheter receiving chamber between the base sheet and the cover sheet. The seal between the base sheet and the cover sheet may be an adhesive or a heat weld formed with the material of the base sheet and cover sheet. The base sheet of material and the cover sheet of material may each define a projecting end section thereon located outboard of the seal with respect to the catheter receiving chamber, with the projecting end sections not connected to each other and with the opening initiating means located in the projecting end sections of the base and cover sheets outboard of the seal. The lubricant may extend from that end of the catheter chamber at the projecting end of the catheter for about 1/4-1/2 the length of the chamber and the viscosity range of the lubricant may be selected so that the lubricant does not significantly flow along the length of the catheter at normal storage temperature but readily flows at normal body temperature. The guide means may be located within the package so that the projecting end of the catheter is located within the catheter receiving opening defined by the guide means when the catheter receiving chamber is sealed and the guide means may be constructed and arranged so that the catheter can be gripped through the package at the guide means by manually pressing opposite sides of the package together at the guide means with guide means constructed and arranged to normally maintain the catheter receiving opening at a cross-sectional size and shape larger than the cross-sectional diameter of the projecting end of the catheter when the package is manually released. The guide means may he affixed to the package so that the guide means is axially fixed relative to the package. The guide means may comprise a resilient tubular guide member having opposed ends thereon bonded to the package at said first end so that the tubular guide member lies within the sealed catheter receiving chamber in the package before the package is opened and projects out of the opening in the first end of the package when the package is opened, where the guide member defines the catheter receiving opening therethrough opening onto the opposed ends of the guide member so that the guide member will slidably pass therethrough, and where the guide member normally has a generally circular configuration in its relaxed condition so that the guide member can be manually pressed together to cause the guide member to grip the catheter and the guide member will return toward its normally relaxed condition when released to permit the catheter to pass therethrough. Alternatively, the guide means may comprise a preformed guide strip bonded to one side of the package at the first end so that the guide member lies adjacent the sealed catheter receiving chamber in the package before the package is opened and extends along one side of the opening in the first end of the package when the package is opened with the guide member defining a recess thereacross generally in alignment with the first axis through the package and with that side of the package immediately adjacent the guide member affixed to and extending into the recess to form a conforming recess to the recess in the guide member so that the conforming recess formed in the side of the package has a cross-sectional size and shape to slidably receive the catheter therethrough to define the catheter receiving opening between the sides of the package whereby the guide member and that side of the package opposite the guide member can be manually pressed together to cause the catheter to be gripped through the sides of the package yet the catheter will be released to freely slide through the catheter receiving opening when the guide strip and the opposite side of the package are released to permit the catheter to pass therethrough.

A method of packaging not comprised by the invention as defined by the claims is directed to a method of packaging urinary catheters of a prescribed length and with a projecting end for use in catheterizing patients comprising the steps of: a) forming a primary recess in a base sheet of material sized to receive the catheter therein where the recess has a first end along with a secondary guide recess adjacent the first end to slidably guide the catheter; and, b) depositing a lubricant in the recess so that the lubricant extends from the first end of the recess a prescribed distance toward the opposite end of the recess and so that the lubricant does not overfill the recess when the catheter is also located in the recess. The method of packaging may also include the step of placing one of the catheters in the recess so that the projecting end of the catheter is located at the first end of the recess prior to step b). The method of packaging may also comprise the steps of placing a cover sheet of material over the base sheet of material so that the cover sheet covers the recess; and, sealing the cover sheet to the base sheet around the recess so as to seal the recess with the catheter and lubricant therein. The method of packaging may also comprise the step of forming a weakened section in the base and cover sheets in the vicinity of the first end so that the first end of the package can be manually opened at the weakened section.

The method of using the catheter and package assembly is directed to a method of using a urinary catheter carried within an elongate sealed package where the catheter has a projecting end located at a first end of the sealed package and a discharge end opposite the projecting end, and where guide means is provided within the sealed chamber at the first end to guide the catheter out of the chamber, and lubricant is provided within the package about the projecting end of the catheter comprising the steps of a) making a first opening through the first end of the package of a sufficient size for the projecting end of the catheter to pass therethrough; b) making a second opening through the package in the vicinity of the discharge end of the catheter; and, c) after the first and second openings are made, manually extending the projecting end of the catheter out of the package by manually gripping and releasing the catheter within the chamber through the guide means while manipulating the catheter through the package.

These and other features and advantages of the invention will become more clearly understood upon consideration of the following detailed description and accompanying drawings wherein like characters of reference designate corresponding parts throughout the several views and in which:

### BRIEF DESCRIPTION OF THE DRAWINGS

Fig. 1 is a perspective view of a urinary catheter and package assembly incorporating the invention;
Fig. 2 is an exploded perspective view of the urinary catheter and package assembly of Fig. 1;
Fig. 3A is an enlarged side view of the projecting end of the assembly of Fig. 1;
Fig. 3B is an enlarged side view of the drainage end of the assembly of Fig. 1;
Fig. 4 is an enlarged cross-sectional view taken generally along line 4-4 in Fig. 3A;
Fig. 5 is an enlarged perspective of the guide structure of that embodiment of the invention seen in Fig. 1;
Fig. 6 is an enlarged end view of the guide structure seen in Fig. 4 with the catheter passing through it;
Fig. 7 is a cross-sectional view taken along line 7-7 in Fig. 6 without the catheter in the guide passage;
Fig. 8 is the cross-sectional view of Fig. 7 with the catheter in the guide passage;
Fig. 9 is a cross-sectional view similar to Fig. 7 illustrating a second embodiment of the extension control mechanism;
Fig. 10 is the cross-sectional view of Fig. 9 with the catheter in the guide passage;
Fig. 11A is an enlarged side view of the projecting end of a second embodiment of the invention;
Fig. 11B is an enlarged side view of the drainage end of the invention of Fig. 11B;
Fig. 12 is an enlarged perspective view of the second embodiment of the guide structure shown in Fig. 11;
Fig. 13 is an enlarged perspective view illustrating a third embodiment of the extension control mechanism for use with the second embodiment of the guide structure seen in Fig. 12;
Fig. 14 is an enlarged side view of the third embodiment of the extension control mechanism seen in Figs. 11-13;
Fig. 15 is an enlarged end view of the guide structure of Fig. 11 with the third embodiment of the extension control mechanism being used to control the movement of the catheter;
Fig. 16 is an enlarged cross-sectional view taken along line 16-16 in Fig. 15;
Fig. 17 is an enlarged perspective view illustrating a fourth embodiment of the extension control mechanism for use with the second embodiment of the guide structure seen in Fig. 12;
Fig. 18 is an enlarged side view of the fourth embodiment of the extension control mechanism seen in Fig. 17;
Fig. 19 is an enlarged end view of the guide structure of Fig. 17 with the fourth embodiment of the extension control mechanism being used to control the movement of the catheter;
Fig. 20 is an enlarged cross-sectional view taken along line 20-20 in Fig. 19;
Fig. 21 is an enlarged side view of the first embodiment of the invention seen in Figs. 1 and 2 being assembled;
Fig. 22 is a perspective view of a fifth embodiment of the urinary catheter and package assembly of the invention;
Fig. 23A is an enlarged side view of the projecting end of the assembly of Fig. 22;
Fig. 23B is an enlarged side view of the drainage end of the assembly of Fig. 22;
Fig. 24 is an enlarged cross-sectional view taken generally along line 24-24 in Fig. 23A;
Fig. 25 is an enlarged perspective view of the projecting end of the assembly of Fig. 22 illustrating the package in a sealed condition;
Fig. 26 is a view similar to Fig. 25 illustrating the end of the package having been opened to expose the tip of the catheter;
Fig. 27 is an enlarged perspective view of the projecting end of sixth embodiment of the catheter and package assembly of the invention in a sealed condition;
Fig. 28 is a view similar to Fig. 27 illustrating the catheter and package assembly having been opened to expose the tip of the catheter;
Fig. 29 is a perspective view of the assembly of Fig. 1 illustrating both ends of the catheter being extended from the package;
Fig. 30 is a perspective view of the assembly of Fig. 22 illustrating both ends of the catheter being extended from the package;
Fig. 31 is a perspective view illustrating the package arranged as a drainage extension; and,
Fig. 32 is a schematic view illustrating the fabrication of the fifth embodiment of the catheter and package assembly of the invention.

These figures and the following detailed description disclose specific embodiments of the invention, however, it is to be understood that the inventive concept is not limited thereto since it may be embodied in other forms.

### DETAILED DESCRIPTION OF ILLUSTRATIVE EMBODIMENTS

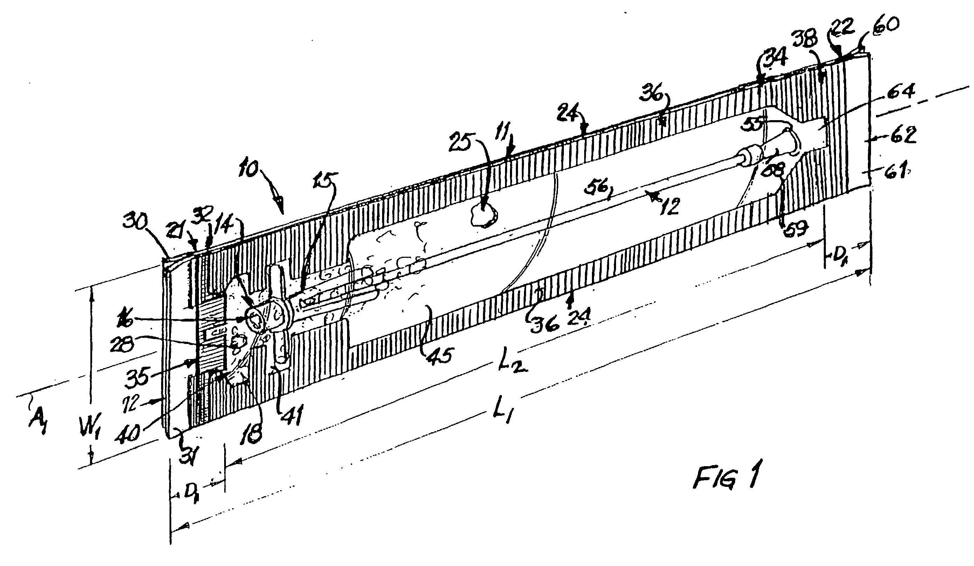

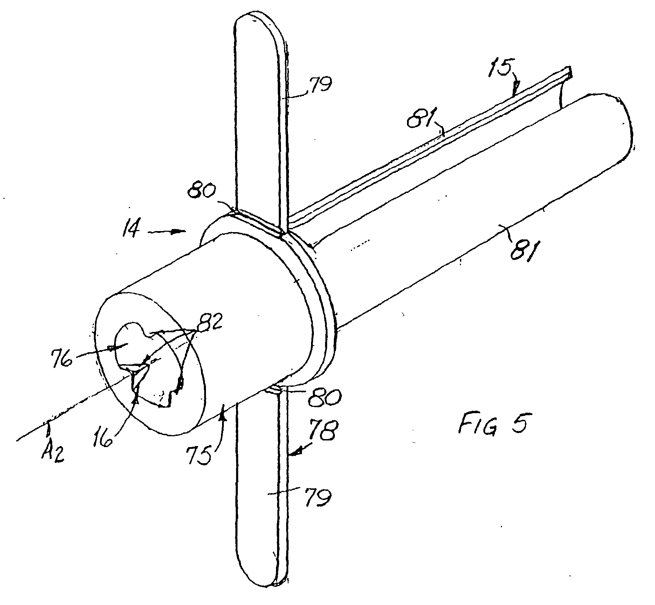

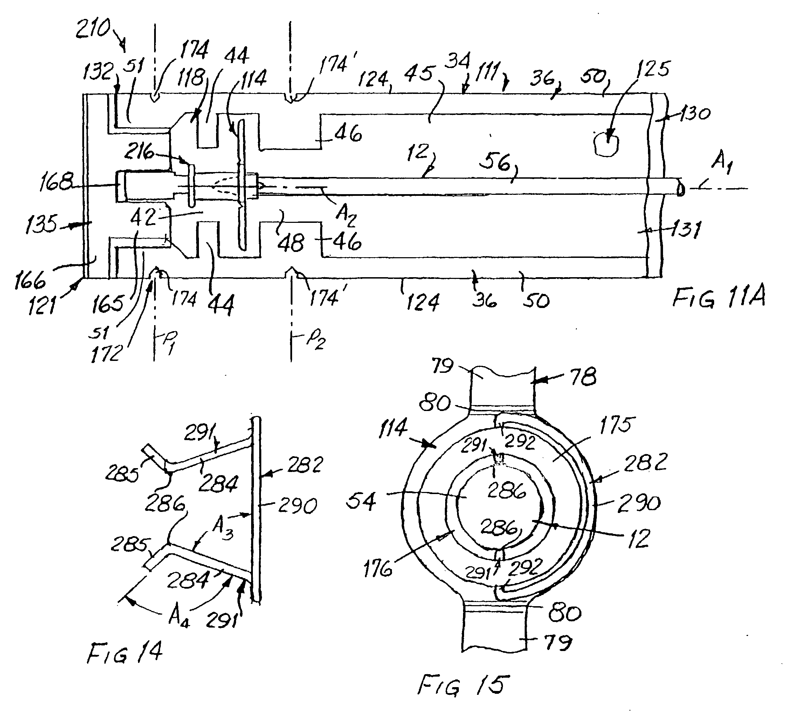

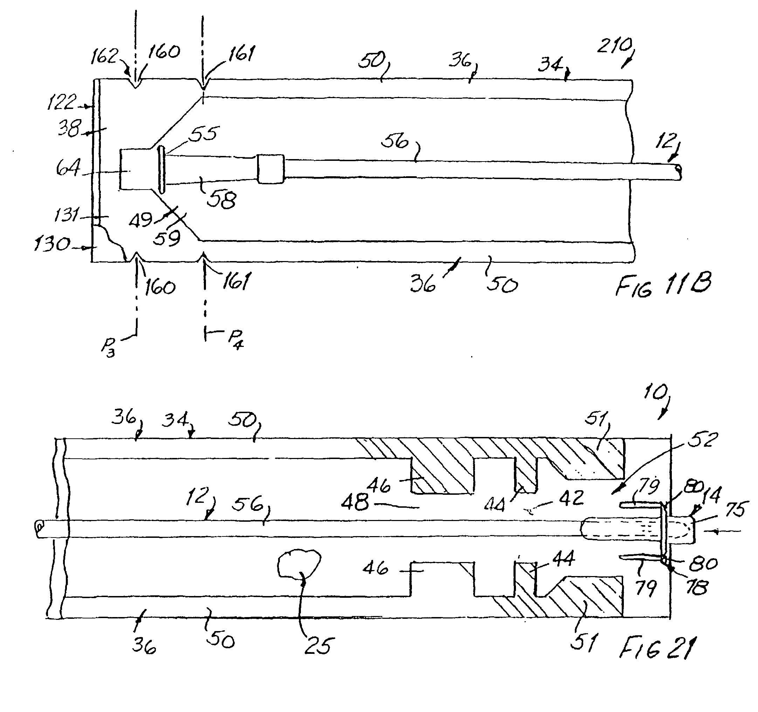

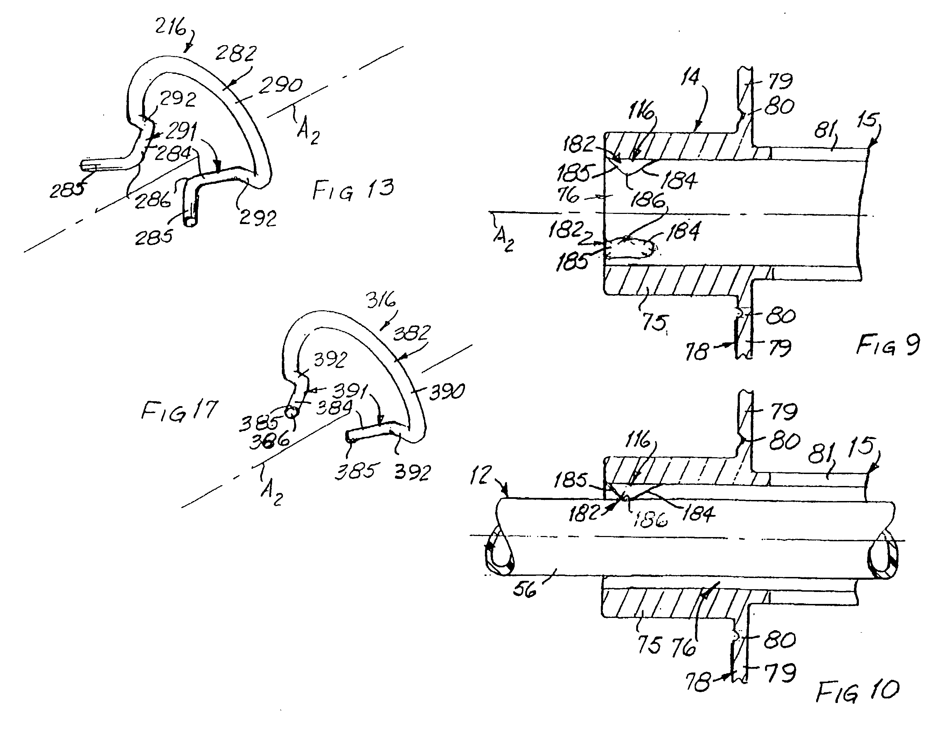

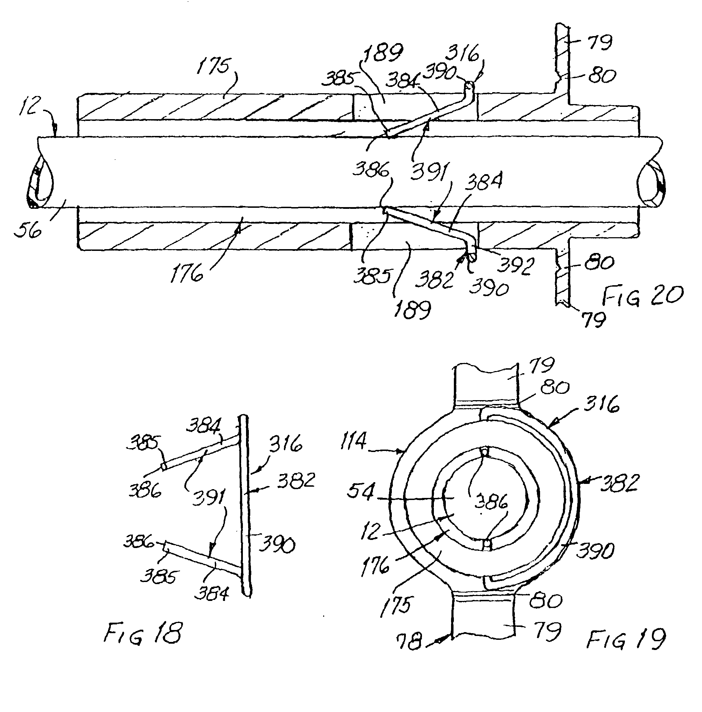

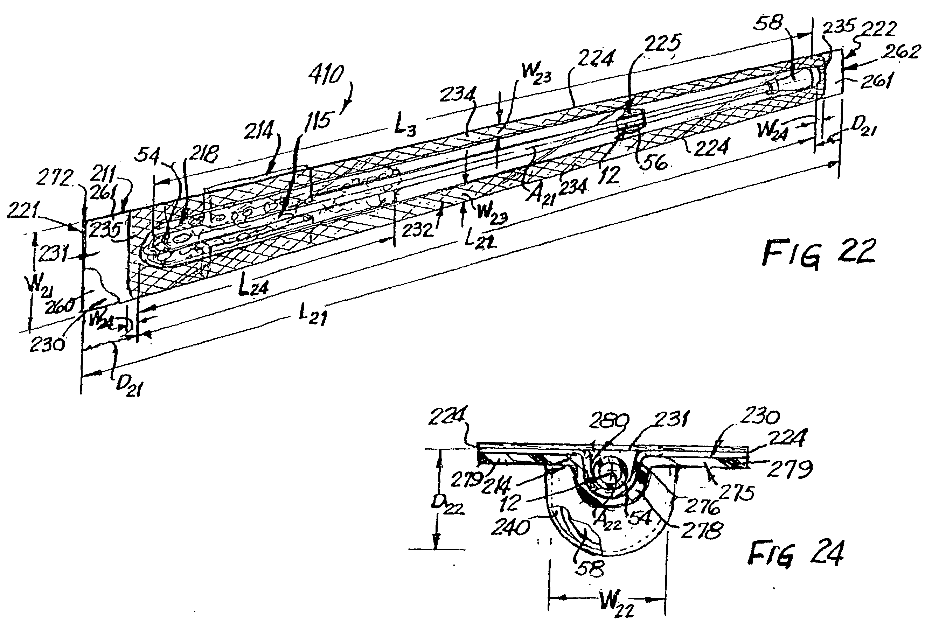

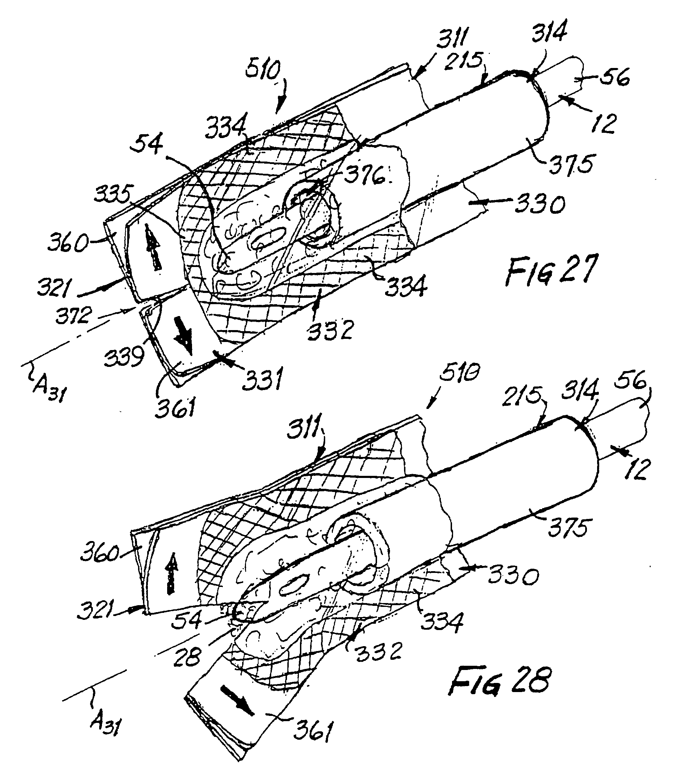

Referring to the drawings, several embodiments of the catheter and package assemblies are shown as well as several embodiments of the package, guide means, gripping means, extension control means, and lubricating means. A first embodiment 10 of the catheter and package assembly is illustrated in Figs. 1-8, a second embodiment is illustrated in Figs. 9-10, a third embodiment 210 is illustrated in Figs. 11-16, a fourth embodiment is illustrated in Figs. 17-20, a fifth embodiment 410 is illustrated in Figs. 22-26, and a sixth embodiment 510 is illustrated in Figs. 27 and 28. A first embodiment 11 of the package is illustrated in Figs. 1-10 and 20, a second embodiment 111 of the package is illustrated in Figs. 11-19, a third embodiment 211 is illustrated in Figs. 22-26, and a fourth embodiment 311 is illustrated in Figs. 27 and 28. A first embodiment 14 of the guide means is illustrated in Figs. 1-3A and 4-10, a second embodiment 114 of the guide means is illustrated in Figs. 11-18, a third embodiment 214 of the guide means is illustrated in Figs. 22-26, and a fourth embodiment 314 of the guide means is illustrated in Figs. 27 and 28. A first embodiment 15 of the gripping means is illustrated in Figs. 1-10, a second embodiment 115 is illustrated in Figs. 22-26, and a third embodiment 215 is illustrated in Figs. 27-28. A first embodiment 16 of the extension control means is illustrated in Figs. 1-8, a second embodiment 116 is illustrated in Figs. 9 and 10, a third embodiment 216 is illustrated in Figs. 11-16, and a fourth embodiment 316 is illustrated in Figs. 17-20. A first embodiment 18 of the lubricating means is illustrated in Figs. 1-8, a second embodiment 118 is illustrated in Figs. 11-13, and a third embodiment 218 is illustrated in Figs. 22-31.

Turning to Figs. 1-4, it will be seen that the catheter and package assembly 10 includes package 11, a catheter 12 carried in the package 11, guide means 14 adjacent one end of the package 11, gripping means 15 on the guide means 14, extension control means 16 also on the guide means 14, and lubricating means 18 around a portion of the catheter 12 in the package 11. The guide means 14 helps the user guide the catheter 12 as it is extended from the package 11 while the gripping means 15 facilitates the user gripping the catheter 12 through the package for manipulating the catheter within the package. The extension control means 16 serves to prevent the catheter 12 from being inadvertently retracted back into the package 11 when the package is moved relative to the catheter for extension from the package. The lubricating means 18 serves to prelubricate the catheter 12 so that the catheter 12 is ready to use as it is extended from the package 11.

The package 11 has an elongate rectilinear shape with opposed first and second end portions 21 and 22 respectively and opposed side portions 24. The package 11 has an overall length L₁ and an overall width W₁ as best seen in Fig. 1. The package 11 defines a sealed elongate catheter receiving chamber 25 therein about the longitudinally extending first axis A₁ through the package. The sealed elongate catheter receiving chamber 25 extends along the first axis A₁ from the first end portion 21 to the second end portion 22 so that the chamber 25 has a minimum prescribed length L₂ as will become more apparent. Thus, the package 11 projects beyond the chamber 25 on both ends for a projection distance D₁ also seen in Fig.1.

The package 11 is formed with a base sheet 30 and a cover sheet 31 of material which is at least semiflexible and which does not allow contaminates to pass therethrough to maintain the catheter 12 in a sterile condition until ready for use. Preferably, at least one of the sheets 30 and 31 is made of flexible transparent or translucent plastic of the type commonly used in the medical products industry to maintain medical products sterile. A number of different materials may be used for one or both sheets 30 and 31 without departing from the scope of the invention. The kind of materials will also depend on the method by which the package 11 is to be opened as will become more apparent. The sheets 30 and 31 are illustrated with common lengths L₁ and common widths W₁.

The catheter receiving chamber 25 is formed between the sheets 30 and 31 with the sheets 30 and 31 sealed together around the chamber 25 with a composite seal indicated generally at 32. The seal can be formed by any convenient means such as with a heat activated adhesive for the seal 32 or by heat sealing the sheets directly to each other as illustrated in Fig. 11 for the seal 132. Typically, where the package 10 is to be opened by peeling the sheets 30 and 31 apart as illustrated in Figs. 1-4, a heat activated strippable adhesive is used to form the seal 32 with the material commonly used for the sheets 30 and 31 being a coextrusion of polyvinylchloride (PVC) and polyethylene. Where the package is to be opened by tearing as illustrated for the package 111, a polyethylene is typically used with an oriented grain for the sheets 130 and 131. The sheets 130 and 131 are heat sealed directly to each other. The polyethylene material is such that, once a tear is started, the sheets will tear straight across the package to form a straight edge at the tear. It will be appreciated that the seals 32 or 132 can be interchanged with each other without departing from the scope of the invention.

As seen in Figs. 1-4, the composite seal 32 comprises a primary package forming seal 34 and a closing seal 35. The primary package forming seal 34 is used to form the basic package 11 but leaves the catheter receiving chamber open at the first end portion 21 of the package so that the catheter 12 and guide means 14 can be loaded into the package. The closing seal 35 serves to seal the catheter receiving chamber 25 after the catheter and guide are loaded.

The package forming seal 34 has opposed side sections 36 that extend along opposite sides of the package 11 and an end section 38 at the second end portion 22 of the package 11 joining the side sections 36 to close the catheter receiving chamber 25 at the second end portion of the package. The closing seal 35 joins the side sections 36 at the first end portion 21 of the package 11 to close the catheter receiving chamber.

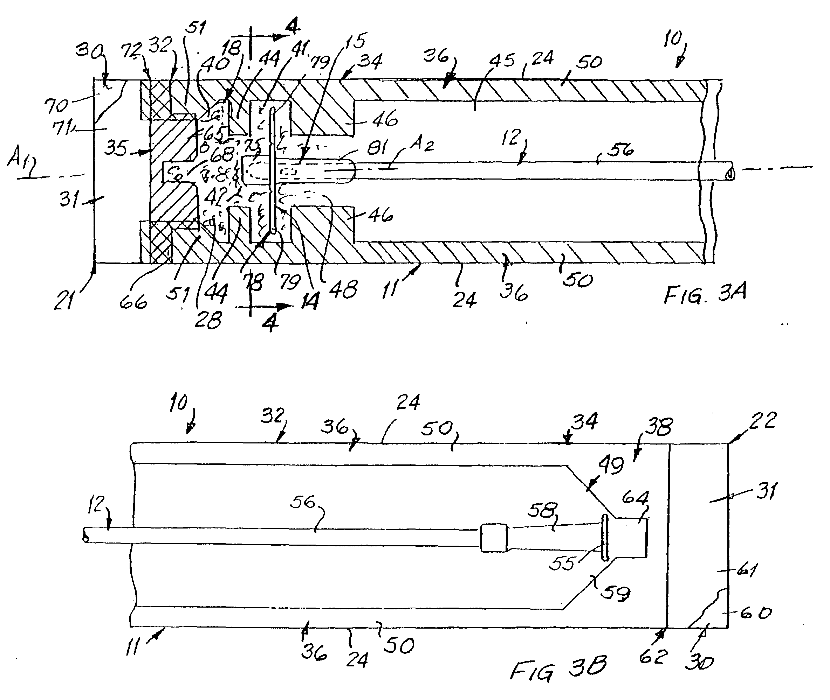

As best seen in Figs. 3A and 3B, the catheter receiving chamber 25 has different coaxially arranged subchambers to maintain the catheter 12 and guide means 14 in place within the package 11. A lubricant subchamber 40 of chamber 25 is formed in the package 11 at the first end portion 21 and forms part of the lubricating means 18. A guide retaining subchamber 41 of chamber 25 is formed in the package adjacent the lubricant subchamber 40 and communicates with the subchamber 40 through a smaller central opening 42 formed by first separation projections 44 in the side sections 36 of seal 34. A catheter subchamber 45 of chamber 25 is formed in the package 11 adjacent that side of the guide retaining subchamber 41 opposite the lubricant subchamber 40 and communicates with the subchamber 41 through a smaller central opening 46 formed by second separation projections 48. The end section 38 of the primary seal 34 forms a discharge subchamber 49 of chamber 25 in the package 11 at that end of the catheter subchamber 45 opposite the guide retaining subchamber 41. The discharge subchamber 49 forms an extension of the catheter subchamber 45 and projects into the second end portion 22 of the package 11.

Each side section 36 of the primary seal 34 has a side portion 50 which extends along the length of the chamber 25 with the first and second separator projections 44 and 46 projecting inwardly therefrom. Third separator projections 51 form a portion of the lubricant subchamber 40 on that side of the subchamber 40 opposite the guide retaining subchamber 41. The projections 51 define a loading opening 52 therebetween through which the catheter 12 and guide means 14 are loaded into the package 11 as will become more apparent.

The size of the lubricant subchamber 40 is determined by the amount of lubricant needed for application to the catheter 12 as will become apparent. The guide retaining subchamber 41 is sized so that the guide means 14 will be captivated therein as will become more apparent. The catheter subchamber 45 is sized to locate the catheter 12 within the guide means 14 as will become more apparent. The discharge subchamber 49 is sized and configured to direct urine out of the chamber 25 when the package 11 is used as an extension to the catheter 12 and to prevent the catheter 12 from inadvertently dropping out of the package 11 when the end portion 22 is opened as will become more apparent. The loading opening 52 as well as the central openings 42 and 46 are sized so that the guide means 14 in a collapsed condition can pass therethrough for loading as will become more apparent.

The end section 38 joins the side portions 50 of the side sections 36 at the second end portion 22 of the package 11 and extends out from the end of the chamber 25 a distance less than the projection distance D₁ so that the sheets 30 and 31 project beyond the ends of the chamber 25 to form nonattached projecting end portions 60 and 61 in the sheets 30 and 31 respectively outboard of the end section 38 of the seal 34. The end portions 60 and 61 form part of the second opening initiating means 62 and are adapted to be manually gripped to facilitate the opening of the discharge end of the package 11 as will become apparent.

As best seen in Figs. 1 and 3A, the closing seal 35 serves to close the loading opening 52 between the separator projections 51 to seal the catheter receiving chamber 25. The closing seal 35 extends across the full width of the package 11 and is provided with a confining projection 65 that extends from the base band 66 of the seal 35 toward the lubricant subchamber 40. The projection 65 defines a recess therein forming a reduced diameter passage 68 centered on axis A, and opening into the lubricant subchamber 40. The opposite end of the passage 68 is initially closed by the base band 66 to maintain the catheter receiving chamber 25 sealed. As will become more apparent, the closed end of the passage 68 is opened when the user opens the package 11 to allow the catheter 12 to move out through the passage 68.

The closing seal 35 extends out from the end of the chamber 25 a distance less than the projection distance D₁ so that the sheets 30 and 31 project beyond the ends of the chamber 25 at the first end portion 21 to form nonattached projecting end portions 70 and 71 in the sheets 30 and 31 respectively outboard of the closing seal 35. The end portions 70 and 71 form part of the first opening initiating means 72 and are adapted to be manually gripped to facilitate the opening of the first end portion 21 of the package 11 as will become apparent.

The opening initiating means 62 and 72 are designed to facilitate the manual opening of the ends of the catheter receiving chamber 25 as seen in Figs. 1-3. The opening initiating means 62 and 72 are designed to make the package 11 easy to open manually for persons who may have limited manual dexterity and to confine the opening to the ends of the package 11. To open the ends of the catheter receiving chamber 25, the user grasps the unattached end portions 60 and 61 or 70 and 71 and pulls the sheets 30 and 31 apart until the seal 32 has been peeled back far enough to open the end of the chamber 25 as will become more apparent.

The catheter 12 corresponds to those catheters currently in use for intermittent catheterization. Catheter 12 has a projecting end 54 and an opposed drainage end 55, and includes an elongate catheter tube 56 typically equipped with an enlarged funnel 58 at the drainage end 55. The tip of the catheter tube 56 at the projecting end 54 is closed and equipped with an inlet opening adjacent the tip to the drain passage that extends from the inlet opening along the length of the catheter tube 56 and out of the end of the funnel 58. The length L₃ of the catheter 12 is determined by the physical size of the patient with which the catheter is to be used. A typical length for catheters 12 designed for use with both men and women is about 17-18 inches. The length L₃ thus determines the length L₂ of the catheter receiving chamber 25 which must be longer than the length of the catheter 12 so that the catheter 12 will be stowed in the package 11 in a straight condition. The catheter tube 56 is round and the funnel 58 is also typically round. The funnel 58 has the largest cross-sectional dimension D₃ while the catheter tube 56 has a smaller diameter D₄. The cross-sectional diameter D₄ varies depending on the size of the catheter and typically ranges from 6-22 French.

The discharge subchamber 49 formed in the chamber 25 has a conical portion 59 therein at the end of the catheter subchamber 45 that tapers down to a discharge passage 64 coaxially aligned with the axis A₁. The discharge passage 64 has a diameter less than the funnel diameter D₃ so that the funnel 58 will not pass out of the discharge passage. Initially, the outer end of the discharge passage 64 is closed by the end section 38 of the seal 34 but is opened when the second end portion 22 of the package 11 is opened as will become more apparent. The passage 64 allows the urine to drain out of the package 11 when it is used as a drainage extension as will become more apparent. If the user wants to use the package as a drainage extension, the user grasps the unattached end portions 60 and 61 and pulls the sheets 30 and 31 apart until the end section 38 of the primary seal 34 has been peeled back only far enough to open the end of the passage 64. If, on the other hand, the user wants to slide the package 11 up over the funnel 58 to allow the urine to drain directly out of the discharge end 55 of the catheter, the user continues to pull the sheets 30 and 31 apart to peel back the end section 38 of the primary seal 34 until the conical portion 59 has been opened sufficiently for the end portion 22 of the package 11 to pass over the funnel 58.

The guide means 14 best seen in Figs. 1-8 assists the user in being able to control the position of the catheter tube 56 as it is extended out of the package 11. Because the cross-sectional size of the recess catheter receiving chamber 25 is considerably larger than that of the catheter tube 56, the catheter tube 56 tends to slip around within the chamber 25 as it is being extended from the package. The guide means 14 is located at that end of the package 11 at which the projecting end 54 on catheter 12 is located and serves to guide the catheter tube 56 as it is being extended to facilitate the user controlling the catheter tube. As best seen in Fig. 5, the guide means 14 includes a cylindrical tubular guide member 75 having a central axis A₂ and defining a guide passage 76 therethrough concentrically about axis A₂ with a diameter large enough for the catheter tube 56 to freely pass therethrough but smaller than the diameter of the funnel 58 to prevent its passage therethrough. The material for the guide member 75 is selected so that the guide member is substantially rigid.

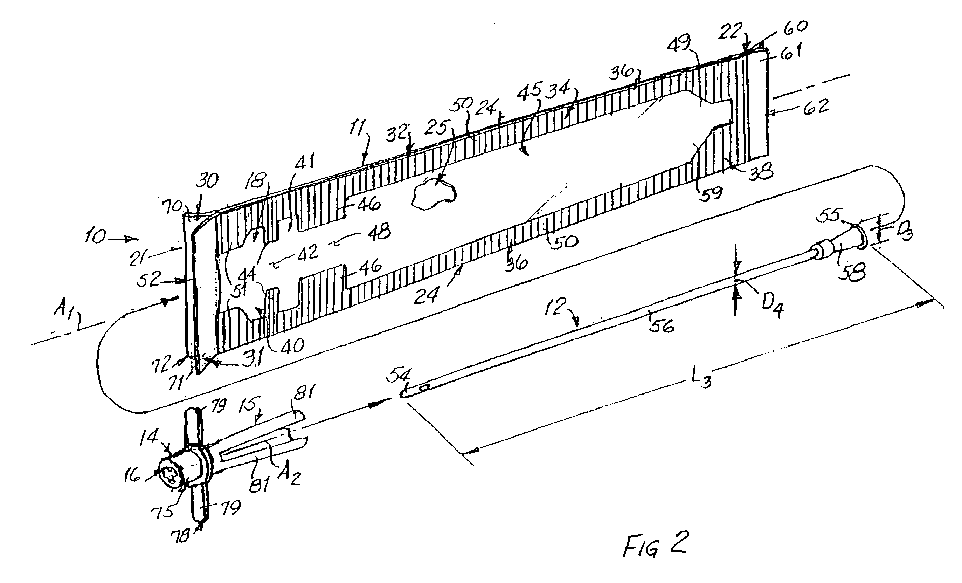

A guide holding arrangement 78 is provided on the member 75 for use in interconnecting the guide means 14 to the package 11 to keep the guide means 14 fixed relative to a portion of the package. The arrangement 78 includes a pair of diametrically outwardly projecting flanges 79 joined to the guide member 75 through a bendable joint 80 so that the flanges 79 can be moved between an erected position in which the flanges 79 are oriented normal to the axis A₂ as seen in Figs. 1 and 2 and a stowed position in which the flanges 79 are bent over to a position generally parallel to the axis A₂ as seen in Fig. 21. This allows the guide means 14 to be inserted into the chamber 25 through the loading opening 52 as illustrated in Fig. 21 with the flanges 79 stowed and then bent outwardly with an appropriate erection tool (not shown) after the flanges 79 become aligned with guide retaining subchambers 41 so that the flanges 79 are captivated in the subchambers 41 and thus locate the guide means 14 relative to the package 11 with the guide axis A₂ in alignment with the package axis A, as seen in Figs. 1 and 2.

The gripping means 15 best seen in Fig. 5 is mounted on the guide means 14 and projects outwardly therefrom along the guide axis A₂. The gripping means 14 includes a pair of opposed, spaced apart gripping flanges 81 flexibly connected to that end of the guide member 75 facing the catheter subchamber 45. The flanges 81 have a convenient length to permit the user to press inwardly on the flanges through the package 11 to grip the catheter 12 between the flanges 81 to positively lock the catheter 12 to the gripping means 15. When the flanges 81 are released, the natural resiliency of the material from which the flanges 81 are formed moves them back apart to release the catheter tube 56.

The extension control means 16 best seen in Figs. 5-8 is also mounted on the guide means 14. The extension control means 16 is a one-way clutch which permits the catheter 12 to be extended from the package 11 but substantially prevents the catheter 12 from being retracted back into the package 11 once extended. The extension control means 16 comprises a plurality of check members 82 on the inside of the guide member 75 and projecting into the guide passage 76. While different numbers of check members 82 may be used without departing from the scope of the invention, three are illustrated for extension control means 16.

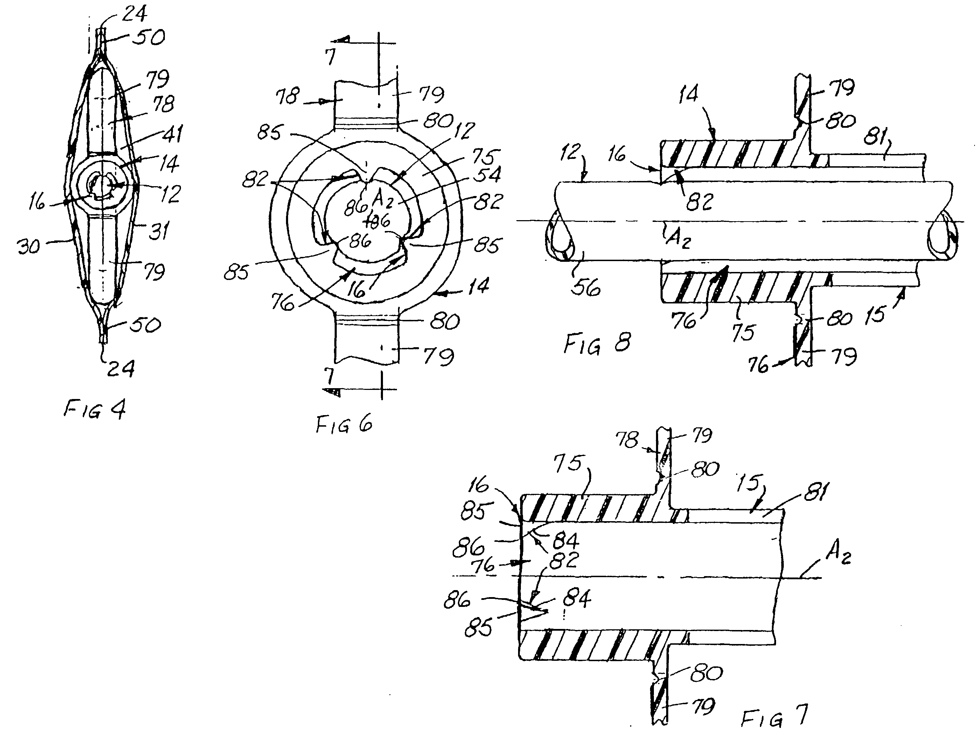

The check members 82 are equally spaced around the inside of the guide member 75 and are arranged in a common plane normal to the guide member axis A₂. Each of the check members 82 includes a sloped surface 84 that faces the catheter subchamber 45 when the guide means is installed in the package 11 and a normal surface 85 that faces in the opposite direction to the sloped surface 84. The surfaces 84 and 85 intersect to form a check edge 86 projecting into the guide passage 76. The height H₁ of each of the check members 82 is selected so that the check edges 86 subtend a circle inside the guide passage 76 of diameter D₅ as best seen in Fig. 7. The diameter D₅ is selected so that it is smaller than the smallest catheter tube diameter D₄ with which the extension control means is to be used. The check members 82 are illustrated as substantially rigid so that the check edges 86 are tightly forced against the surface of the catheter tube 56 due to the natural resiliency of the catheter tube maintaining the tube forced against the edges 86. The sloped surfaces 84 allow the catheter tube 54 to slide past the check members 82, however, the normal surface 85 serves to cause the check edges 86 to bite into the catheter tube and prevent it from being retracted back into the package 11. This facilitates the use of the catheter 12 because the user does not have to worry about the catheter being withdrawn as that portion of the package 11 spaced from the guide means 14 is shifted relative to the catheter 12 so that the catheter can be inserted further by again grasping the catheter through the package and advance the catheter relative to the guide means 16.

It will be appreciated that different sizes of the check members 82 may be necessary to accommodate the different size ranges of catheter tubes 56 although one size of check members 82 may accommodate more than one size of catheter tube depending on the resiliency of the catheter tube itself. Moreover, it will be appreciated that the check members 82 may be fabricated so that they are sufficiently resilient to accommodate a broader range of sizes for the catheter tubes 56 without departing from the scope of the invention.

It will also be appreciated that the extension control means 16 should remove the minimum amount of lubricant from the surface of the catheter tube 56. The check members 82 accomplish this by having narrow rounded check edges 86 as best seen in Fig. 6 so that the contact between the check members 82 and the catheter tube 56 is minimized.

The lubricating means 18 best seen in Figs. 1-3A comprises the lubrication subchamber 40 and a lubricant 28 in the subchamber 40. The closing seal 35 closes the subchamber 40 leaving the passage 68 when the first end of the package 11 is opened. Because the sheets 30 and 31 are flexible, the user can either deliberately squeeze the package 11 at the subchamber 40 or inherently squeeze the package 11 in the vicinity of the subchamber 40 as the catheter 12 is being extended. This forces the lubricant 28 around the catheter tube 56 as it passes through the subchamber 40 to insure that the lubricant coats the catheter tube 56. Because each portion of the catheter must pass through the lubrication subchamber 40 after it passes the extension control means 16, any lubricant 28 removed from the tube 56 is replenished before the catheter tube 56 passes out of the package 11 during use.

The second embodiment of the catheter and package assembly utilizes those components of the first embodiment except a second embodiment of the extension control means is utilized. Figs. 9 and 10 illustrate the second embodiment 116 of the extension control means. The extension control means 116 is also mounted on the guide means 14. The extension control means 116 will allow movement of the catheter 12 in either direction through the guide means 14 but serves to prevent movement of the catheter 12 relative to the guide means 14 until a minimum axial force is applied to the catheter tube 56. The threshold value of the force required move the catheter tube 56 relative to the guide means 14 is selected sufficiently high so that the package can be moved relative the catheter tube without moving the catheter tube 56 with respect to the guide means 14 yet sufficiently low so that the catheter tube 56 can be moved relative to the guide means 16 when the catheter tube 56 is grasped through the package 11 to advance or retract the catheter tube.

The extension control means 116 comprises a plurality of limit members 182 on the inside of the guide member 75 and projecting into the guide passage 76. While different numbers of limit members 182 may be used without departing from the scope of the invention, three are also illustrated for extension control means 116.

The limit members 182 are also equally spaced around the inside of the guide member 75 and are arranged in a common plane normal to the guide member axis A₂. Each of the limit members 182 includes a first sloped surface 184 that faces the catheter subchamber 45 when the guide means is installed in the package 11 and a second sloped surface 185 that faces in the opposite direction to the sloped surface 184. The surfaces 184 and 185 intersect to form a rounded retention edge 186 projecting into the guide passage 76. The height H₁ of each of the limit members 82 is likewise selected so that the retention edges 186 subtend a circle inside the guide passage 76 of diameter D₅. The diameter D₅ is selected so that it is smaller than the smallest catheter tube diameter D₄ with which the extension control means is to be used. The limit members 182 are also illustrated as substantially rigid so that the retention edges 186 are tightly forced against the surface of the catheter tube 56 due to the natural resiliency of the catheter tube maintaining the tube forced against the edges 186. The sloped surfaces 184 and 185 allow the catheter tube 54 to slide in either direction past the limit members 182 when the threshold value is exceeded in the pushing force on the catheter tube 56. This also facilitates the use of the catheter 12 because the user does not have to worry about the catheter being withdrawn as that portion of the package 11 spaced from the guide means 14 is shifted relative to the catheter 12 but the catheter tube 56 can be extended or retracted by grasping the catheter through the package and pushing on the catheter tube with a force exceeding the threshold value.

It will be appreciated that different sizes of the limit members 182 may be necessary to accommodate the different size ranges of catheter tubes 56 although one size of limit members 82 may accommodate more than one size of catheter tube depending on the resiliency of the catheter tube itself. Moreover, it will be appreciated that the limit members 182 may be fabricated so that they are sufficiently resilient to accommodate a broader range of sizes for the catheter tubes 56 without departing from the scope of the invention.

Like the check members 82, the limit members 182 also should remove the minimum amount of lubricant from the surface of the catheter tube 56. The limit members 182 also accomplish this by having narrow rounded retention edges 186 so that the contact between the limit members 182 and the catheter tube 56 is minimized.

The third embodiment 210 of the catheter and package assembly is shown in Figs. 11-16. The assembly 210 includes the same catheter 12 but the second embodiment 111 of the package is used along with a second embodiment 114 of the guide means and a third embodiment 216 of the extension control means. Also a second embodiment 118 of the lubricating means is used.

The package 111 has sheets 130 and 131 joined together by the seal 132 to form the catheter receiving chamber 125 therein. As explained hereinabove, the seal 132 is a heat weld directly bonding the sheets 130 and 131 together. The sheets 130 and 131 are made out of the oriented polyester material so that it will tear straight as will become more apparent. The catheter receiving chamber 125 has the same guide retaining subchamber 41 and catheter subchamber 45 as the first embodiment of the package. The seal 132 has the same primary package forming seal 34 as the first embodiment of the package. The closing seal 135 extends to the end of the package 111 as best seen in Fig. 11A with the band 166 extending the full width of the package and the confining projection 165 extends to the close the lubricant subchamber 140. The projection 165 also defines a recess therein but the reduced diameter passage 168 centered on axis A₁ and opening into the lubricant subchamber 140 is sized to receive the guide means 114 as will become more apparent.

The second embodiment 172 of the first opening initiating means on the package 111 is a pair of notches 174 cut into opposite sides of the package 111 at the path P₁ at which the first end portion 121 is to be torn to open the package. The material of sheets 130 and 131 is such that the package 111 will tear straight across from either one of the notches 174. The first pair of notches 174 is located so that the package 111 will tear across the passage 168 to open it and expose the guide means 114 as will become more apparent. In the event the user wants to use the catheter 12 without the guide means 114, the opening initiating means 172 also includes a second pair of notches 174' located on opposite sides of the package 111 along path P₂. Path P₂ extends across the projections 46 so that the package 111 will tear to release the guide means 114 so that it can be removed from the catheter 12. The second embodiment 162 of the second opening initiating means on the package 111 is also a pair of notches 160 cut into opposite sides of the package 111 at the path P₃ normal to axis A₁ at which the second end portion 122 is to be torn to open the discharge end of the package. The package 111 will thus tear straight across from either one of the notches 170. The first pair of notches 160 is located so that the package 111 will tear across the passage 64 to open it and allow urine to drain from the catheter subchamber 45 while preventing the catheter 12 from inadvertently falling out of the package 111. In the event the user wants to expose the funnel 58 to allow the urine to drain directly out of the discharge end 55 of the catheter, a second set of notches 161 are provided. The notches 161 are located on opposite sides of the package 111 along path P₄. Path P₄ extends across the side sections 36 at the juncture of the end section 38 of the primary package forming seal 34 so that the package 111 will tear to open up the end of the catheter subchamber 45 to release the funnel 58 so that the end portion 22 of the package 111 to pass over the funnel 58.

The second embodiment 114 of the guide means also includes a cylindrical tubular guide member 175 having a central axis A₂ and defining a guide passage 176 therethrough concentrically about axis A₂ with a diameter large enough for the catheter tube 56 to freely pass therethrough but smaller than the diameter of the funnel 58 to prevent its passage therethrough. The material for the guide member 175 is also selected so that the guide member is substantially rigid. The guide tube 176, however, is longer than that of guide member 75 so that the guide member will extend across the lubricant subchamber 140 into the passage 168. It will be seen that the length of the guide member 175 is such that the projecting end 188 of the member 175 extends past the path P₁ so that the end 188 of the guide member 175 will project out of the package 111 when the package is torn along the path P₁. The catheter tube 56 can exit the package 111 through the now exposed end of the guide member 175 so that the catheter is not exposed to a contaminated surface as it extends from the package 111.

The guide means 114 uses the same guide holding arrangement 78 as the first embodiment 14. The guide holding arrangement 78 is located adjacent that end of the guide member 175 facing the catheter subchamber 45 as seen in Fig. 11A.

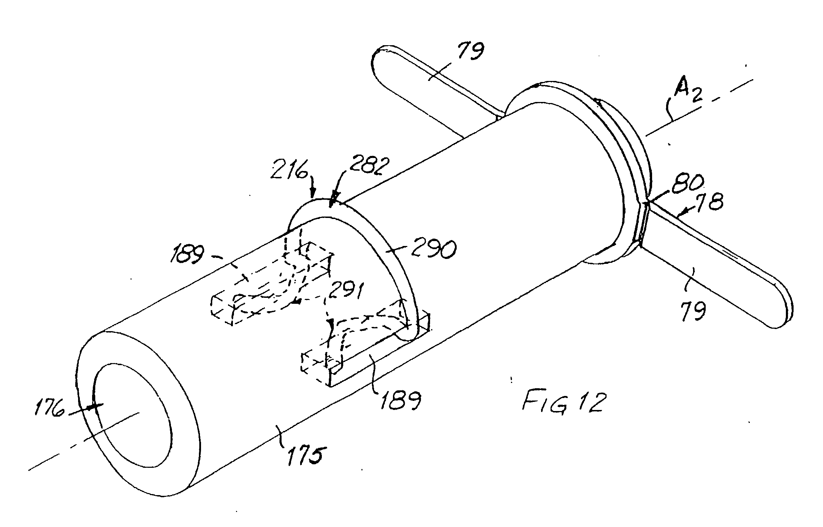

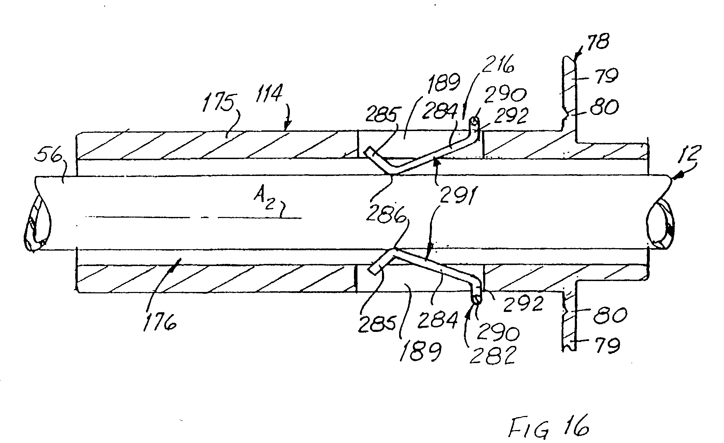

The guide member 175 defines a pair of diametrically opposed slots 189 through the side wall thereof as best seen in Figs. 12 and 16. The slots 189 are located in registration with the lubricant subchamber 140 so that lubricant in the subchamber 140 can flow into the guide passage 176 to coat the catheter tube 56 passing therethrough as will become more apparent. The slots 189 also function as part of the extension control means 216 as will become more apparent.

The extension control means 216 as best seen in Figs. 12-16 includes a resilient retention member 282 that projects through the slots 189 in the guide member 175 into the guide passage 176 to engage the catheter tube 56 passing through the guide member 175. The retention member 282 is a spring member with a semi-circular base section 290 that extends around the outside of the guide member 175 between the slots 189. Opposite ends of the base section 290 are provided with inwardly projecting retention sections 291 that extend through the slots 189 into the guide passage 176. Each of the sections 291 includes a short positioning leg 292 seen in Figs. 13 and 16 that bears against the edge of the slot 189 to check the movement of the retention member 282 toward the catheter subchamber 45. The legs 292 lie in the same plane as the base section 290. Each of the retention sections 291 also include a first sloped leg 284 integral with the projecting end of the positioning leg 292 that angles inwardly from the plane of the base section 291 and leg 292 with a prescribed included angle A₃ seen in Fig. 14. The projecting end of the first sloped leg 284 is integral with a second sloped leg 285 through a curved section 286. The second sloped leg 285 defines a prescribed included angle A₄ with the first sloped leg 284 seen in Fig. 14 so that the curved section 286 will bear against the catheter tube 56 passing through the guide passage 176 and the projecting end of the leg 285 will extend back into the slot 189. The spacing distance D₅ between the curved sections 286 as seen in Fig. 14 is less than the outside diameter D₄ of the catheter tube 56 so that the curved sections 286 will resiliently bear against the outside of the catheter tube 56. The spring constant of the retention member 282 is selected so that the minimum threshold value of the axial force necessary to move the tube 56 relative to the retention member 282 is greater than the force exerted on the catheter tube 56 when that portion of the package 111 along the catheter subchamber 45 spaced away from the guide means 114 is moved relative to the catheter tube as is necessary when the package and catheter are manipulated with respect to each other during extension of the catheter 12 from the package 111. On the other hand, the minimum threshold value is less than the axial force which can be reasonably exerted on the catheter tube 56 when the user manually grips the catheter tube 56 through the package 111 even though the catheter tube may be coated with lubricant.

It will be seen that the retention member 282 can be designed to accommodate a wide range of catheter tube sizes. Further, because of the relative small cross-sectional diameter of the member 282, any removal of the lubricant from the surface of the catheter tube 56 as it passes the member 282 is minimized.

The lubricating means 118 also comprises the lubricant subchamber 140 with lubricant 28. The subchamber 140 is substantially closed around the guide member 175 so that, when the package 111 is manually pressed in the vicinity of the lubricant subchamber 140, the lubricant 28 will be forced through the slots 189 in the guide member 175 into the guide passage 176 around the catheter tube 56. This insures that the catheter tube will be well lubricated for use.

The fourth embodiment of the catheter and package assembly includes a fourth embodiment 316 of the extension control means best seen in Figs. 17-20. The extension control means 316 also includes a resilient retention member 382 that projects through the slots 189 in the guide member 175 into the guide passage 176 to engage the catheter tube 56 passing through the guide member 175. The retention member 382 is a spring member with a semi-circular base section 390 like the base section 290 of the third embodiment of the extension control means. Opposite ends of the base section 390 are provided with inwardly projecting retention sections 391 that extend through the slots 189 into the guide passage 176. Each of the sections 391 includes a short positioning leg 392 seen in Figs. 17 and 20 that bears against the edge of the slot 189 to check the movement of the retention member 382 toward the catheter subchamber 45. The legs 392 lie in the same plane as the base section 390. Each of the retention sections 391 also includes a sloped leg 384 integral with the projecting end of the positioning leg 392 that angles inwardly from the plane of the base section 390 and leg 392 with the prescribed included angle A₃ seen in Fig. 18. The projecting end 385 of the sloped leg 384 has a check end surface thereon normal to the leg axis. This forms a check edge 386 that allows the catheter tube 56 to slide past the retention sections 391 out of the catheter subchamber 45 but bites into the surface of the catheter tube if the catheter tube tries to retract back into the catheter subchamber 45.

It will be seen that the retention member 382 can be designed to accommodate a wide range of catheter tube sizes. Further, because of the relative small cross-sectional diameter of the member 382, any removal of the lubricant from the surface of the catheter tube 56 as it passes the member 382 is minimized.

The fifth embodiment 410 of the catheter and package assembly is seen in Figs. 22-26. The catheter and package assembly 410 includes a third embodiment 211 of the package, the catheter 12, a third embodiment 214 of the guide means, a second embodiment 115 of the gripping means, and a third embodiment 218 of the lubricating means.

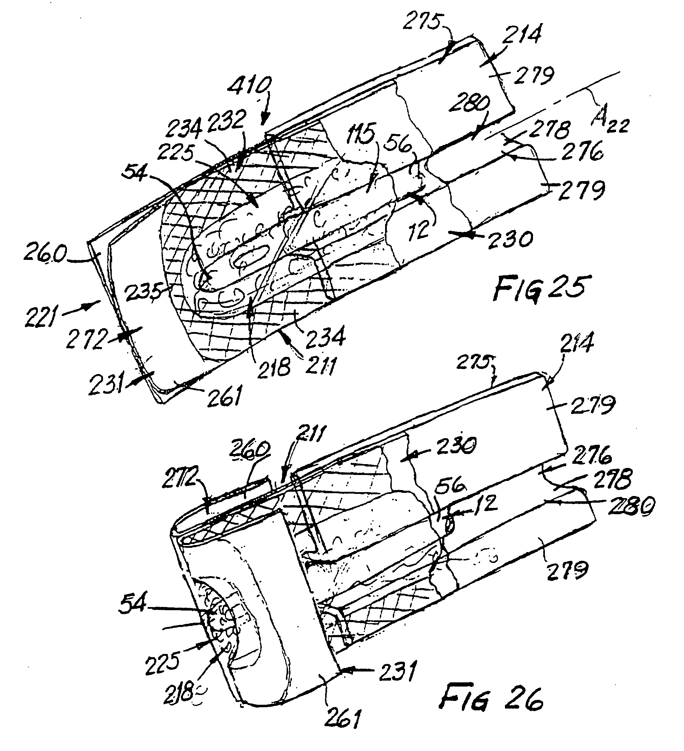

The package 211 also has an elongate rectilinear shape with opposed first and second end portions 221 and 222 and opposed side portions 224. The package 211 has an overall length L₂₁ and an overall width W₂₁ as best seen in Fig. 22. The package 211 defines sealed elongate catheter receiving chamber 225 therein about the longitudinally extending first axis A₂₁. The catheter receiving chamber 225 extends along the first axis A₂₁ from the first end section 221 to the second end section 222 so that the chamber 225 has a minimum length L₂₂ corresponding to the length of the catheter 12. Thus, the package 211 projects beyond the catheter receiving chamber 225 for projection distance D₂₁ seen in Fig. 22. As will become more apparent, the catheter receiving chamber 225 has a cross-sectional size and shape as seen in Fig. 24 larger than the maximum diameter D₃ of the funnel 58 so that the package 211 can be pulled along the length of the catheter 12 for the package 211 to act as a drainage extension.

The package 211 is formed of the base sheet 230 and cover sheet 231 of a material similar to that of the first embodiment of the package. The sheets 230 and 231 also have the same length and width as the package 211. The sheets 230 and 231 are heat sealed with a strippable heat seal 232 that extends around the catheter receiving chamber 225. The heat seal 232 has side sections 234 along the sides of the chamber 225 that extend from the side edge of the chamber 225 to the adjacent side portion 224 of the package 211. The heat seal 232 also includes end sections 235, each of which joins the side sections 234 and projects out from the end of the chamber 225 a distance less than the projection distance D₂₁ so that the sheets 230 and 231 project beyond the ends of the chamber 225 to form nonattached projecting end portions 260 and 261 in opposite end portions 221 and 222 of the sheets 230 and 231 respectively. The end portions 260 and 261 are adapted to be manually gripped to peel the ends of the package 211 apart like the package 11. The end portions 260 and 261 at the first end portion 221 of the package 211 thus form the first opening initiating means 272 while the end portions 260 and 261 at the second end portion 222 of the package 211 form the second opening initiating means 262.

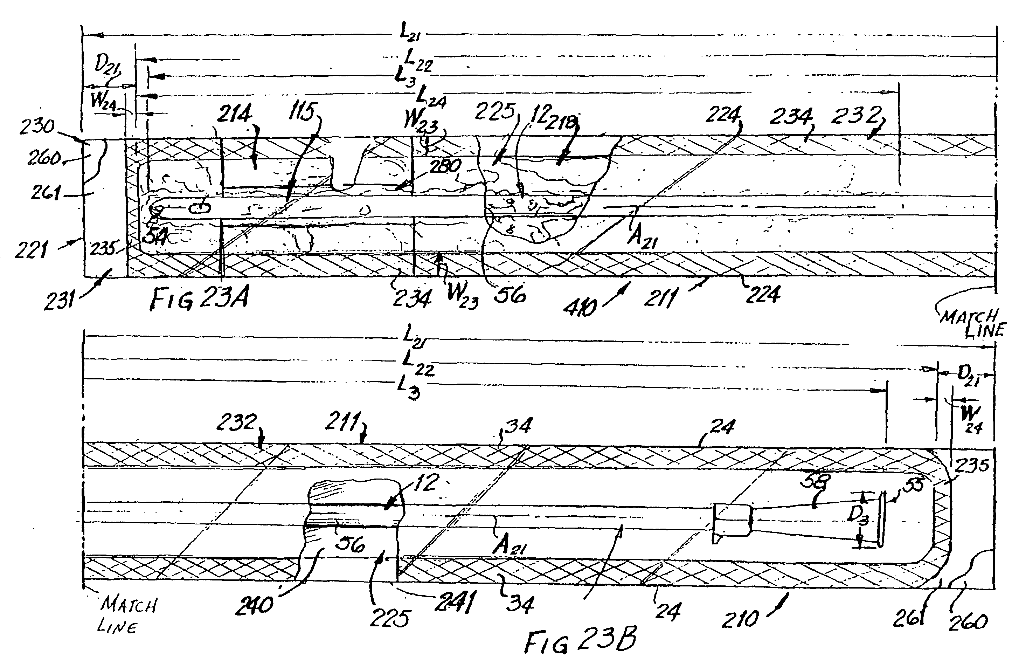

In the embodiment shown, the base sheet 230 has a preformed recess 240 formed therein with the length L₂₁ as seen in Fig. 22, an interior depth D₂₂ as best seen in Fig. 24, and an interior width W₂₂ also best illustrated in Fig. 24. As will become more apparent, the depth D₂₂ and width W₂₂ are selected to be larger than the maximum cross-sectional dimension of the catheter 12 so that all of the catheter will lie within the confines of the recess 240 as seen in Fig. 4. While the particular interior cross-sectional shape of the recess 240 may be varied to suit the particular maximum exterior cross-sectional shape of the catheter 12, the recess 240 illustrated has a U-shape with a rounded bottom. While not meant to be limiting, the recess 240 is illustrated as having a substantially constant cross-sectional shape along the major portion of its length except at the guide means 214. The recess 240 opens onto that side of the base sheet 230 facing the cover sheet 231 to form a loading opening 241 seen in Figs. 23B, 25 and 26 through which the catheter 12 and the lubricant are loaded into the recess 240.

The cover sheet 231 spans the recess 240 and is generally planar in the embodiment illustrated. Those juxtaposed portions of the sheets 230 and 231 along opposite sides of the package 211 are heat sealed to form the side sections 234 of the seal 232 with a transverse width W₂₃ as seen in Fig. 23A. On the other hand, only that part of the juxtaposed portions of the sheets 230 and 231 adjacent the catheter receiving chamber 225 at opposite ends of the package 211 are heat sealed to form the end sections 235 of the seal 232 with a transverse width W₂₄ as seen in Figs. 23A and 23B. The width W₂₄ is less than the width W₂₃ so that it is easier to peel apart the end sections 235 than the side sections 234 for the opening of the chamber 225 to usually be confined to the ends thereof as will become more apparent.

The guide means 214 on the catheter and package assembly 410 is located at that end of the package 211 at which the projecting end 54 on catheter 12 is located and serves to guide the catheter tube 56 as it is being extended to facilitate the user controlling the catheter tube. While the guide means may be embodied in a variety of forms without departing from the scope of the invention, it is illustrated as a guide strip 275 seen in Figs. 22-26.

The guide strip 275 extends across the package 211 adjacent the first end 221 thereof and serves to guide the catheter tube 56 as it is being extended by the user. The guide strip 275 has a central guide axis A₂₂ defined therethrough which is positioned generally parallel with the longitudinal axis A₂₁ of the package 211 as seen in Fig. 23A. The strip 275 has a central concave guide section 278 centered on the axis A₂₂ and a pair of opposed side flange sections 279 integral with opposite sides of the guide section 278 and projecting outwardly therefrom so that the sections 279 are coplanar. While different cross-sectional shapes may be used, the guide section 278 illustrated is U-shaped defining a guide recess 276 along the axis A₂₂ with an entrance opening 280 thereto along the length of the recess 276.

The guide strip 275 is secured to the sheet 230 at the first end portion 221 as seen in Fig. 22 so that the recess 276 faces the sheet 230 and the side flange sections 279 lie in juxtaposition with the sheet 230. The sheet 230 is forced into the recess 276 so that a conforming recess is formed in the sheet 230 that opens into the catheter receiving recess 225. The recess 276 is sized so that the catheter tube 56 will be slidably received therein inside the package 211 as seen in the drawings. The opposed sheet 231 spans the entrance opening 280 to keep the catheter tube 56 in place in the recess 276 so that the catheter tube 56 will be guided out of the package 211 by the guide recess 276.

The gripping means 115 comprises the combination of the recess 276 in the guide member 275 and the flexible cover sheet 231. The user can grip the catheter tube 56 in the guide recess 276 by pressing in on the sheet 231 to force the tube 56 against guide member 276 to grip it.

The lubricating means 218 includes the lubricant 28 placed in a particular position within the package 211. The lubricant 28 is that typically used with catheters and is designed to liquify at body temperature. At storage temperature, however, the lubricant 28 is sufficiently viscous so as not to significantly flow along the length of the catheter 12 or chamber 225 in the package 211. While different viscosity parameters may be used without departing from the scope of the invention, a lubricant with a viscosity range of 200,000-400,000 cps at 20-25°C. measured using Brookfield RVT, spindle 6, 2.5 rpm has performed satisfactorily. The lubricant 28 is located in the chamber 225 and extends from that end of the chamber 225 at the first end 221 of the package 211 a distance L₂₄ as best seen in Fig. 22. The length of lubricant is selected such that a sufficient length of the catheter 12 will be lubricated for use. A typical length is 1/4-1/2 the length of the catheter 12. The lubricant 28 is evenly distributed along the distance L₂₄ with a sufficient quantity to adequately coat the catheter 12 but at the same time not sufficient to overfill the recess 240 in combination with the catheter tube 56 so that the lubricant 28 will not be squeezed out of the recess 240 when the cover sheet 231 is applied. While different quantities may be used, about 1-3 grams of lubricant has been found satisfactory.

The sixth embodiment 510 of the catheter and package assembly is illustrated in Figs. 27 and 28. The assembly 510 utilizes a fourth embodiment 311 of the package. The package 311 uses the oriented polyethylene material for sheets 330 and 331 which are heat welded together to form the heat seal 332. The seal 332 has side sections 334 and end sections 335 similar in shape to sections 234 and 235.

Referring to Figs. 27 and 28, the first opening initiating means 372 is a cut 339 through the projecting end portions 360 and 361 on the base and cover sheets 330 and 331. The cut 339 extends from the end edges on the sheets 330 and 331 to the vicinity of the end section 335 of the seal 332 and is generally axially aligned with the first axis A₃₁ of the package 311. To open the end of the catheter receiving chamber 325, the user grasps the end portions 360 and 361 on opposite sides of the cut 339 and pulls outwardly transversely of the axis A₃₁ as indicated by the arrows. This causes the sheets 330 and 331 to tear across the end section 335 of the seal 332 to form the opening to the end of the chamber 325 best seen in Fig. 28 so that the projecting end of the catheter 12 can be extended therethrough.

The assembly 510 includes the fourth embodiment 314 of the guide means and the third embodiment 215 of the gripping means as seen in Figs. 27 and 28. The guide means 314 includes a flexible tubular guide member 375 defining guide passage 376 therethrough. The guide member 375 has enough resilience to recover to its generally circular shape when released as seen in Figs. 27 and 28 but can be squeezed inwardly manually by the user to grip the catheter tube 56 inside the member 375 to hold it relative to the guide member 375. Thus, the guide member 375 also acts as the gripping means 215.

### Using the invention

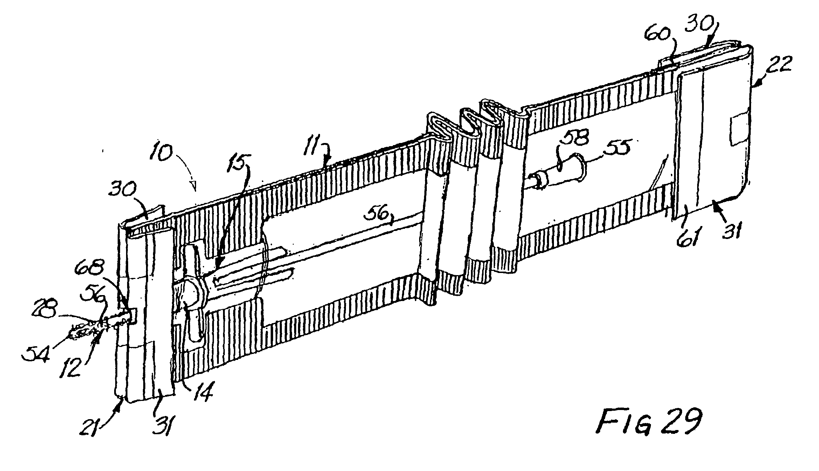

To use the first embodiment of the catheter and package assembly 10, the user opens both ends of the catheter receiving chamber 25 at the opening initiating means 72 and 62 as seen in Fig. 29. This allows the user to extend the projecting end 54 of the catheter 12 out of the passage 68 from the chamber 25 at the first end portion 21 of the package 11 by gripping the catheter 12 through the package 11 and collapsing the package 11 down around the catheter 12 as seen in Fig. 29. Still gripping the catheter 12 through the package 11, the user can insert the catheter 12 through the urethra into the bladder. The extension control means 16 serves to prevent the catheter 12 from being withdrawn back into the package 11 as the package is straightened out along the catheter 12 to allow the user to again grip the catheter 12 through the package 11 and advance the catheter tube 56 further out of the package 11. The user continues to incrementally advance the catheter 12 out of the package 11 until the bladder is reached.

If the user needs to drain the fluid directly out of the drainage end 55 of the catheter 12, the package 11 is pulled back over the funnel 58 as seen in Fig. 29 so that the drainage end 55 passes out through the passage 64 from chamber 25 as seen in Fig. 29. This clears the drainage end 55 from the package 11 so that the fluid can drain directly out of the catheter 12 into a convenient receptacle. On the other hand, if the catheter 12 is not long enough to reach the receptacle to catch the fluid, the user can extend the package 11 over the catheter 12 so that it acts as a drainage extension for the catheter 12. As will become more apparent, the user pulls the package 11 along the catheter 12 while the funnel 58 remains in the chamber 25 so that the package 11 projects out over the discharge end 55 of the catheter 12. In this position, the catheter 12 drains into the chamber 25 in the package 11 and then drains out of the opposite end of the package 11 through the passage 64.

The second embodiment of the catheter and package assembly is used similarly to the first embodiment. It will be appreciated that the extension control means 116 will allow the catheter 12 to be forcibly extended from or retracted into the package 11.

The third embodiment 210 of the catheter and package assembly is used by tearing off the ends of the package 111 starting with one of the notches 174 or 174' at the first end portion 121 of the package and one of the notches 160 or 161 at the second end portion 122. Using the notch 174 tears across the package 111 along the path P₁ so as to expose the end of the guide member 175 to provide an uncontaminated outlet for the catheter 12 to exit the package. The catheter 12 is extended out of the package 111 similarly to the first embodiment 10 of the catheter and package assembly. The extension control means 216 allows the catheter 12 to be extended from or retracted into the package 111.

The fourth embodiment of the catheter and package assembly using the fourth embodiment 316 of the extension control means is used similarly to the third embodiment. The retention member 382 of the extension control means 316 prevents the catheter 12 from being retracted back into the package 111.

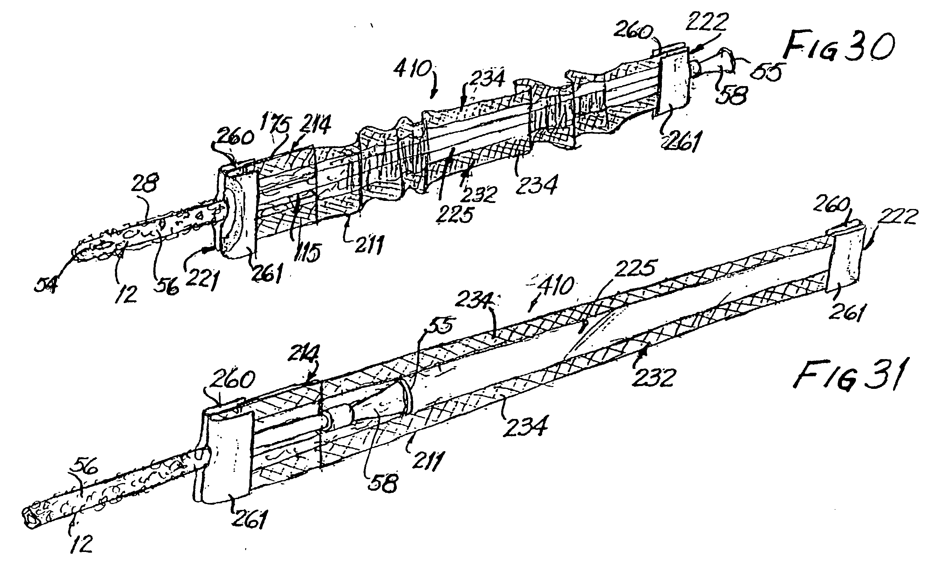

To use the fifth embodiment 410 of the catheter and package assembly, the user opens both ends of the catheter receiving chamber 225 at the opening initiating means 272 and 262 as seen in Fig. 30. This allows the user to extend the projecting end 54 of the catheter 12 out of the chamber 225 at the first end portion 221 of the package 211 by gripping the catheter 12 through the package 211 and collapsing the package 211 down around the catheter 12 as seen in Fig. 30. Still gripping the catheter 12 through the package 211, the user can insert the catheter 12 through the urethra into the bladder. The user grips the catheter 12 at the gripping means 115 to prevent the catheter 12 from being withdrawn back into the package 211 as the package is straightened out along the catheter 12 to allow the user to again grip the catheter 12 through the package 211 and advance the catheter tube 56 further out of the package 211. The user continues to incrementally advance the catheter 12 out of the package 211 until the bladder is reached.

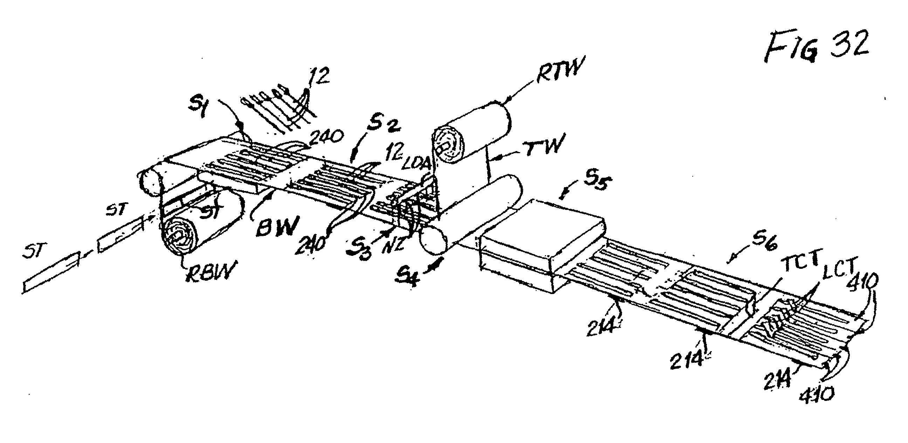

If the user needs to drain the fluid directly out of the drainage end 55 of the catheter 12, the package 211 is pulled back over the funnel 58 as seen in Fig. 30 so that the drainage end 55 passes out of the chamber 225 through the second end portion 222 as seen in Fig. 30. This clears the drainage end 55 from the package 211 so that the fluid can drain directly out of the catheter 12 into a convenient receptacle. On the other hand, if the catheter 12 is not long enough to reach the receptacle to catch the fluid, the user can extend the package 211 over the catheter 12 so that it acts as a drainage extension for the catheter 12 as seen in Fig. 31. The user pulls the package 211 along the catheter 12 while the funnel 58 remains in the chamber 225 so that the package 211 projects out over the discharge end 55 of the catheter 12. In this position, the catheter 12 drains into the chamber 225 in the package 211 and then drains out of the opposite open end portion 222 of the package 211.

### Manufacture

The catheter and package assemblies incorporating the invention may be fabricated in any convenient way without departing from the scope of the invention. For purposes of illustration, the first embodiment 10 of the catheter and package assembly is shown being fabricated by initially making the package 11 with the primary seal 34 leaving the loading opening 52 open as seen in Figs. 2 and 21. The catheter 12 is usually then partly inserted into the guide means 14 as seen in Fig. 21, the flanges 79 of the guide holding arrangement 78 folded back to the stowed position seen in Fig. 21, and the guide means 14 and catheter 12 combination inserted into the package 11 through the loading opening 52. After the guide means 14 reaches the vicinity of the guide retaining subchamber 41 in the package, the flanges 79 are erected back to their erected position as seen in Fig. 1 so that the flanges 79 extend into and are captivated in the subchamber 41 to fix the guide means 14 with respect to that portion of the package 11. The closing seal 35 is then made to seal the package 11.

The second embodiment of the catheter and package assembly uses the package 11 and is fabricated like the first embodiment. The third embodiment 210 of the catheter and package assembly uses a package 111 having a similar construction as the package 11 and this embodiment is also fabricated similarly to the first embodiment 10 of the catheter and package assembly. The fourth embodiment of the catheter and package assembly uses the package 111 and is fabricated like the third embodiment.

The fifth embodiment 410 of the catheter and package assembly is illustrated being fabricated on a vacuum packaging machine. Fig. 32 illustrates the fabrication of the assembly 410 schematically.

As seen in Fig. 32, the packages 211 of the assembly 410 are fabricated from a bottom web BW from which the base sheets 230 are cut and a top web TW from which the cover sheets 231 are cut. The bottom web BW is unwound from a roll RBW and strips ST which will form the guide strips 275 are attached to the web BW at spaced apart positions corresponding to the location of the strip on each completed package 211. The thusly made web and strips passes through a vacuum forming station S₁, a catheter loading station S₂, a lubricant loading station S₃, a cover sheet application station S₄, a sealing station S₅, and cutting station S₆. While different numbers of assemblies 410 can be simultaneously made without departing from the scope of the invention, five assemblies 410 are illustrated as being made at once in Fig. 32. The bottom web BW is indexed through the stations S₁-S₆ sequentially stopping in those stations requiring processing while the web is stopped.

In the vacuum forming station S₁, the recesses 240 are vacuum formed in the web BW in conventional manner while simultaneously forming the recesses 276 in the strips ST. The web BW with the preformed recesses 240 is then indexed to the catheter loading station S₂ where the catheters 12 are typically manually loaded into the recesses 240 while the catheters 12 are in a substantially straight condition and with the projecting end 54 of the catheter leading.

The lubricant loading station S₃ is provided with a lubricant dispensing arrangement LDA having a plurality of nozzles NZ, one nozzle NZ in alignment with each recess 240 in the bottom web BW as the bottom web BW passes thereunder while it is being indexed through the station S₃. The lubricant 28 is dispensed from the nozzles NZ in synchronization with the movement of the bottom web BW past the nozzles so that the desired amount of lubricant will be deposited onto the catheter 12 in the recess 240 and evenly distributed from that end of the recess at the projecting end of the catheter for the prescribed distance L₂₄. The cross-sectional size of the lubricant 28 and the catheter tube 54 combination at the point where lubricant is present is less than the cross-sectional size of the interior of the recess 240 so that no lubricant will be squeezed out of the recess 240 in between the juxtaposed portions of the sheets 230 and 231 when the top web TW is applied over the bottom web BW.

As the bottom web BW with the catheters 12 and lubricant 28 loaded in the recesses 240 is indexed to the sealing station S₅, it passes through the cover sheet application station S₄ where the top web TW from the roll RTW is applied over the bottom web BW so that the top web TW is in lateral and longitudinal registration with the bottom web BW. After the top web TW is applied, the top and bottom web combination is moved to the sealing section S₅ where the seals 232 around the recesses 240 are formed. In the particular process illustrated, the seals 232 are formed by heat sealing the sheets 230 and 231 together with a conventional heat sealing device adapted to form the strippable adhesive seal 232 as set forth hereinabove. Alternatively, using polyethylene for the webs BW and TW will allow the sheets 230 and 231 to be heat welded together at the station S₅ to form the seal 232.

The sealed bottom and top webs BW and TW are then indexed into the cutting station S₆ where the opening initiating means 272 and 262 are formed for the individual assemblies 410 and the individual assemblies 410 cut apart for use. As the sealed webs TW and BW move through the cutting station S₆, they pass under the longitudinal cutters LCT which slit the webs lengthwise and then the transverse cutter TCT cuts the packages 211 apart to complete the fabrication process.

To form the sixth embodiment 510 of catheter and package assemblies seen in Figs. 27 and 28, the opening initiating means 372 and 362 can be formed as part of the fabrication shown in Fig. 32. At the cutting station S₆, the cut 339 forming the opening initiating means may be made with an opening initiating cutter properly aligned to makes the cut 339 for both the first opening initiating means 372 and the second opening initiating means 362 at the same time.

## Claims

1. A prelubricated urinary catheter and package assembly for use in draining the bladder through the urethra, the prelubricated urinary catheter being extendible from the package, and the package comprising a first and second end portion, wherein the prelubricated urinary catheter and package assembly comprises:
a) a flexible package defining a catheter receiving chamber therein;
b) a catheter received in the catheter receiving chamber the catheter having a drainage end portion and a projecting end portion for extension from the catheter receiving chamber in the package;
c) a prescribed quantity of lubricant; and
d) lubricating means for causing the lubricant to be applied to the projecting end portion of the catheter for lubricating the catheter for use,
**characterised in that** the prelubricated urinary catheter and package assembly comprises extension control means permitting extension of the catheter from the package, the extension control means being capable of substantially preventing retraction of the catheter back into the package.

2. The prelubricated urinary catheter and package assembly of Claim 1 wherein the extension control means includes gripping means permitting extension of the catheter from the package wherein the gripping means can be operated to substantially prevent retraction of the catheter back into the package.

3. The prelubricated urinary catheter and package assembly of either one of Claims 1 and 2 wherein the lubricating means is constructed and arranged to cause the lubricant to be forced onto the catheter as the catheter is extended out of the package.

4. The prelubricated urinary catheter and package assembly of any one of Claims 1 to 3 further comprising guide means including a preformed guide member defining a urinary catheter guide passage therethrough, the package defining a longitudinally extending first axis therethrough.

5. The prelubricated urinary catheter and package assembly of Claim 4 wherein:
i) the package has the catheter receiving chamber defined therein in a sealed, elongated form, extending along the first axis from the first end portion to the second end portion and having a minimum prescribed length;
ii) the catheter having opposed ends and a maximum length less than the minimum prescribed length of the catheter receiving chamber in the package, the catheter defining a drainage passage extending from the projecting end portion to the drainage end portion, the drainage passage defining an inlet opening thereto at the projecting end portion and a discharge opening therefrom at the drainage end portion so that fluids in the bladder can drain therethrough when the projecting end portion of the catheter is inserted into the bladder through the urethra; the catheter carried in the catheter receiving chamber in a generally straight condition with the projecting end portion of the catheter located at a first end portion of the catheter receiving chamber so that the projecting end portion of the catheter can pass out of the catheter receiving chamber when the first end portion of the catheter receiving chamber is manually opened; wherein the extension control means prevents retraction of the catheter into the catheter receiving chamber; and
iii) the guide means being operatively associated with the first end portion of the package and defining a urinary catheter guide passage within the catheter receiving chamber conforming generally in size and shape to the projecting end portion of the catheter and oriented generally along the first axis through the package, the urinary catheter guide passage communicating with the catheter receiving chamber and operatively associated with the first end portion of the package so that the urinary catheter guide passage remains sealed until the first end portion of the package is manually opened whereupon the projecting end portion of the catheter can slidably pass out of the catheter receiving chamber through the urinary catheter guide passage to guide the catheter out of the catheter receiving chamber.

6. The prelubricated urinary catheter and package assembly of any preceding Claim wherein the package further includes:
a) first opening initiating means at the first end portion of the package for controlling the manual opening of the catheter receiving chamber at the first end portion of the package, and
b) second opening initiating means at the second end portion of the package for controlling the manual opening of the catheter receiving chamber at the second end portion of the package.

7. The prelubricated urinary catheter and package assembly of either one of Claims 4 and 5 wherein the guide means is located within the catheter receiving chamber in the package so that the projecting end portion of the catheter is maintained within the urinary catheter guide passage when the catheter receiving chamber is sealed.

8. The prelubricated urinary catheter and package assembly of any preceding Claim further comprising a gripping arrangement constructed and arranged so that the catheter can be selectively manually gripped within the package for manipulating the catheter and selectively extending the catheter from the package.

9. The prelubricated urinary catheter and package assembly of any one of Claims 4, 5 and 7 wherein the extension control means permits the catheter to move in a first direction through the urinary catheter guide passage so that the projecting end portion of the catheter can be selectively manually pushed out of the package through the urinary catheter guide passage, the extension control means further holding the catheter against movement in a second direction through the urinary catheter guide passage opposite the first direction to substantially prevent the projecting end portion of the catheter from being inadvertently retracted back into the catheter receiving chamber in the package as the catheter is manually manipulated in the package to selectively extend the catheter from the package.

10. The prelubricated urinary catheter and package assembly of any one of Claims 4, 5, 7 and 9 wherein the extension control means controls the movement of the catheter through the urinary catheter guide passage to allow the projecting end portion of the catheter to be selectively manually pushed out of the package through the urinary catheter guide passage, the extension control means including holding means for substantially preventing movement of the catheter through the urinary catheter guide passage until an axial force greater than a prescribed minimum threshold level is applied to the catheter tending to move the catheter through the urinary catheter guide passage.

11. The prelubricated urinary catheter and package assembly of Claim 10 wherein the prescribed minimum threshold level is selected to be sufficient to allow the flexible package to be moved relative to the catheter in the catheter receiving chamber to allow the catheter to be manually manipulated within the catheter receiving chamber so as to selectively extend the catheter from the package.

12. The prelubricated urinary catheter and package assembly of Claim 9 wherein the extension control means comprises one-way clutch means for permitting the catheter to move in the first direction while substantially preventing movement of the catheter in the second direction.

13. The prelubricated urinary catheter and package assembly of Claim 6 wherein the package is further constructed and arranged so as to define a second opening in the catheter receiving chamber when the second end portion of the package is manually opened through the second opening initiating means, the second opening having a cross-sectional size smaller than the cross-sectional size of the drainage end portion of the catheter to prevent the drainage end portion of the catheter from passing therethrough while permitting urine to drain therethrough.

14. The prelubricated urinary catheter and package assembly of any preceding Claim wherein the package further includes a base sheet of material having a length and width corresponding to the length and width of the package; and a cover sheet of material having a length and width corresponding to the length and width of the package, the base sheet and the cover sheet sealed to each other to form the catheter receiving chamber between the base sheet and the cover sheet.

15. The prelubricated urinary catheter and package assembly of any one of Claims 4, 5, 7 and 9 to 12 wherein the guide means comprises a preformed guide strip bonded to one side of the package adjacent the first end portion so that the guide member lies adjacent the catheter receiving chamber in the package before the package is opened and extends across the opening in the first end portion of the package when the package is opened, the guide strip forming a guide recess thereacross located within the catheter receiving chamber and generally in alignment with the first axis of the package, the guide recess having a cross-sectional size and shape to slidably receive the catheter therethrough so that a catheter receiving opening is defined between the guide recess and the side of the package opposite the guide strip whereby the guide strip and that side of the package opposite the guide strip can be manually pressed towards one another to cause the catheter to be gripped within the package and the catheter will be released to freely slide through the catheter receiving opening when the guide strip and the opposite side of the package are released to permit the catheter to pass therethrough.

16. The prelubricated urinary catheter and package assembly of Claim 5 wherein the catheter receiving chamber has a cross-sectional size and shape along its length such that the largest cross-section of the catheter will pass therealong so that the package can be pulled along the catheter until the first end portion of the catheter receiving chamber is located adjacent the drainage end portion of the catheter with the drainage end portion of the catheter still located within the catheter receiving chamber whereby the package acts as a draining extension to the catheter.

17. A method of producing the prelubricated urinary catheter and package assembly of any preceding claim comprising the steps of:
a) forming a primary recess in a base sheet of material sized to receive the catheter therein where the primary recess has a first end and a smaller secondary guide recess within the primary recess adjacent the first end of the primary recess sized to slidably receive the projecting end portion of the catheter therethrough;
b) placing the catheter in the primary and secondary recesses so that the projecting end portion of the catheter is located at the first end of the primary recess and lies within the secondary recess;
c) depositing a lubricant in the primary and secondary recesses so that the lubricant extends from a first end of the primary and secondary recesses a prescribed distance towards the opposite end of the primary and secondary recesses and so that the lubricant does not overfill the primary and secondary recesses when the catheter is located in the primary and secondary recesses;
d) placing a cover sheet of material over the base sheet of material so that the cover sheet covers the primary and secondary recesses; and,
e) sealing the cover sheet to the base sheet around the primary and secondary recesses so as to form a sealed catheter receiving chamber with the catheter and lubricant therein.

18. A method of using the prelubricated urinary catheter and package assembly of any one of Claims 1 to 16 comprising the steps of:
a) making a first opening through the first end portion of the package sufficient for the projecting end portion of the catheter to pass therethrough;
b) making a second opening through the package in the vicinity of the discharge opening of the catheter; and
c) after the first and second openings are made, manually extending the projecting end portion of the catheter out of the package by manually gripping and releasing the catheter in the urinary catheter guide passage through the package and manipulating the catheter through the package.

19. The method of Claim 18 wherein the catheter is not completely removed from the package.

20. The method of either one of Claims 18 and 19 wherein the projecting end portion of the catheter is provided in a straight condition.

## Patentansprüche

1. Ein dauergeschmierter Hamblasenkatheter und Verpackungssystem zur Verwendung beim Drainieren der Blase durch die Harnröhre, wobei der dauergeschmierte Harnblasenkatheter aus der Verpackung ausfahrbar ist, und wobei die Verpackung einen ersten und zweiten Endabschnitt beinhaltet, wobei der dauergeschmierte Harnblasenkatheter und das Verpackungssystem Folgendes beinhalten:
a) eine biegsame Verpackung, die eine Katheter aufnehmende Kammer darin definiert;
b) einen in der Katheter aufnehmenden Kammer aufgenommenen Katheter, wobei der Katheter einen Drainageendabschnitt und einen vorspringenden Endabschnitt zum Ausfahren aus der Katheter aufnehmenden Kammer in der Verpackung aufweist;
c) eine vorgeschriebene Menge an Schmiermittel; und
d) schmierende Mittel, um zu bewirken, dass das Schmiermittel auf den vorspringenden Endabschnitt des Katheters aufgetragen wird, um den Katheter zum Einsatz zu schmieren,
**dadurch gekennzeichnet, dass** der dauergeschmierte Harnblasenkatheter und das Verpackungssystem ein Ausfahrsteuerungsmittel beinhalten, das das Ausfahren des Katheters aus der Verpackung ermöglicht, wobei das Ausfahrsteuerungsmittel das Einfahren des Katheters zurück in die Verpackung im Wesentlichen verhindern kann.

2. Dauergeschmierter Harnblasenkatheter und Verpackungssystem gemäß Anspruch 1, wobei das Ausfahrsteuerungsmittel ein Greifmittel umfasst, das das Ausfahren des Katheters aus der Verpackung ermöglicht, wobei das Greifmittel betätigt werden kann, um das Einfahren des Katheters zurück in die Verpackung im Wesentlichen zu verhindern.

3. Dauergeschmierter Harnblasenkatheter und Verpackungssystem gemäß einem der Ansprüche 1 und 2, wobei das schmierende Mittel so konstruiert und angeordnet ist, dass es bewirkt, dass das Schmiermittel beim Ausfahren des Katheters aus der Verpackung auf den Katheter getrieben wird.

4. Dauergeschmierter Harnblasenkatheter und Verpackungssystem gemäß einem der Ansprüche 1 bis 3, die ferner ein Führungsmittel, einschließlich eines vorgeformten Führungselements, das einen Hamblasenkatheter-Führungsdurchlass dort hindurch definiert, beinhalten, wobei die Verpackung eine sich dort hindurch der Länge nach erstreckende erste Achse definiert.

5. Dauergeschmierter Harnblasenkatheter und Verpackungssystem gemäß Anspruch 4, wobei:
i) die Verpackung die Katheter aufnehmende Kammer darin in abgedichteter, verlängerter Form definiert, wobei sie sich entlang der ersten Achse von dem ersten Endabschnitt zu dem zweiten Endabschnitt erstreckt und eine minimale vorgeschriebene Länge aufweist;
ii) der Katheter, der entgegengesetzte Enden und eine maximale Länge, die weniger als die minimale vorgeschriebene Länge der Katheter aufnehmenden Kammer in der Verpackung ist, aufweist, wobei der Katheter einen Drainagedurchlass definiert, der sich von dem vorspringenden Endabschnitt zu dem Drainageendabschnitt erstreckt, wobei der Drainagedurchlass an dem vorspringenden Endabschnitt eine Einlauföffnung zu diesem, und an dem Drainageendabschnitt eine Ablassöffnung aus diesem definiert, so dass Fluide in der Blase dort hindurch drainiert werden können, wenn der vorspringende Endabschnitt des Katheters durch die Harnröhre in die Blase eingeführt wird; der Katheter in der Katheter aufnehmenden Kammer in einem im Allgemeinen geraden Zustand zu dem vorspringenden Endabschnitt des Katheters, der an einem ersten Endabschnitt der Katheter aufnehmenden Kammer befindlich ist, getragen wird, so dass der vorspringende Endabschnitt des Katheters aus der Katheter aufnehmenden Kammer austreten kann, wenn der erste Endabschnitt der Katheter aufnehmenden Kammer manuell geöffnet wird; wobei das Ausfahrsteuerungsmittel das Einfahren des Katheters in die Katheter aufnehmende Kammer verhindert; und
iii) die Führungsmittel mit dem ersten Endabschnitt der Verpackung betriebsfähig verknüpft sind und einen Harnblasenkatheter-Führungsdurchlass innerhalb der Katheter aufnehmenden Kammer definieren, der im Allgemeinen in der Größe und Form dem vorspringenden Endabschnitt des Katheters entspricht und im Allgemeinen entlang der ersten Achse durch die Verpackung ausgerichtet ist, wobei der Harnblasenkatheter-Führungsdurchlass mit der Katheter aufnehmenden Kammer kommuniziert und mit dem ersten Endabschnitt der Verpackung betriebsfähig verknüpft ist, so dass der Harnblasenkatheter-Führungsdurchlass abgedichtet bleibt, bis der erste Endabschnitt der Verpackung manuell geöffnet wird, wonach der vorspringende Endabschnitt des Katheters gleitbar aus der Katheter aufnehmenden Kammer durch den Harnblasenkatheter-Führungsdurchlass austreten kann, um den Katheter aus der Katheter aufnehmenden Kammer zu führen.

6. Dauergeschmierter Harnblasenkatheter und Verpackungssystem gemäß einem der vorhergehenden Ansprüche, wobei die Verpackung ferner Folgendes umfasst:
a) einleitende Mittel für eine erste Öffnung an dem ersten Endabschnitt der Verpackung zur Steuerung der manuellen Öffnung der Katheter aufnehmenden Kammer an dem ersten Endabschnitt der Verpackung, und
b) einleitende Mittel für eine zweite Öffnung an dem zweiten Endabschnitt der Verpackung zur Steuerung der manuellen Öffnung der Katheter aufnehmenden Kammer an dem zweiten Endabschnitt der Verpackung.

7. Dauergeschmierter Harnblasenkatheter und Verpackungssystem gemäß Anspruch 4 oder 5, wobei das Führungsmittel innerhalb der Katheter aufnehmenden Kammer in der Verpackung befindlich ist, so dass der vorspringende Endabschnitt des Katheters in dem Harnblasenkatheter-Führungsdurchlass gehalten wird, wenn die Katheter aufnehmende Kammer abgedichtet wird.

8. Dauergeschmierter Harnblasenkatheter und Verpackungssystem gemäß einem der vorhergehenden Ansprüche, die ferner eine Greifanordnung beinhalten, die so konstruiert und angeordnet ist, dass der Katheter in der Verpackung selektiv manuell gegriffen werden kann, um den Katheter zu bedienen und den Katheter aus der Verpackung selektiv auszufahren.

9. Dauergeschmierter Harnblasenkatheter und Verpackungssystem gemäß einem der Ansprüche 4, 5 und 7, wobei das Ausfahrsteuerungsmittel ermöglicht, dass sich der Katheter in eine erste Richtung durch den Harnblasenkatheter-Führungsdurchlass bewegt, so dass der vorspringende Endabschnitt des Katheters selektiv manuell aus der Verpackung durch den Harnblasenkatheter-Führungsdurchlass gestoßen werden kann, wobei das Ausfahrsteuerungsmittel ferner den Katheter gegen die Bewegung in eine zweite Richtung durch den Harnblasenkatheter-Führungsdurchlass, die der ersten Richtung entgegengesetzt ist, hält, um im Wesentlichen zu verhindern, dass der vorspringende Endabschnitt des Katheters versehentlich zurück in die Katheter aufnehmende Kammer in der Verpackung eingefahren wird, wenn der Katheter in der Verpackung manuell bedient wird, um den Katheter selektiv aus der Verpackung auszufahren.

10. Dauergeschmierter Harnblasenkatheter und Verpackungssystem gemäß einem der Ansprüche 4, 5, 7 und 9, wobei das Ausfahrsteuerungsmittel die Bewegung des Katheters durch den Harnblasenkatheter-Führungsdurchlass steuert, um zu ermöglichen, dass der vorspringende Endabschnitt des Katheters selektiv manuell aus der Verpackung durch den Harnblasenkatheter-Führungdurchlass gestoßen wird, wobei das Ausfahrsteuerungsmittel Haltemittel umfasst, um die Bewegung des Katheters durch den Harnblasenkatheter-Führungsdurchlass im Wesentlichen zu verhindern, bis eine axiale Kraft, die größer als ein vorgeschriebener minimaler Schwellenwert ist, auf den Katheter angewandt wird, die dazu neigt, den Katheter durch den Harnblasenkatheter-Führungsdurchlass zu bewegen.

11. Dauergeschmierter Harnblasenkatheter und Verpackungssystem gemäß Anspruch 10, wobei der vorgeschriebene minimale Schwellenwert so gewählt ist, dass er ausreicht, um das Bewegen der biegsamen Verpackung relativ zum Katheter in der Katheter aufnehmenden Kammer zu ermöglichen, um das manuelle Handhaben des Katheters innerhalb der Katheter aufnehmenden Kammer zu ermöglichen, so dass der Katheter selektiv aus der Verpackung ausgefahren wird.

12. Dauergeschmierter Harnblasenkatheter und Verpackungssystem gemäß Anspruch 9, wobei das Ausfahrsteuerungsmittel Einwegkupplungsmittel beinhaltet um zu ermöglichen, dass sich der Katheter in die erste Richtung bewegt, während die Bewegung des Katheters in die zweite Richtung im Wesentlichen verhindert wird.

13. Dauergeschmierter Harnblasenkatheter und Verpackungssystem gemäß Anspruch 6, wobei die Verpackung ferner so konstruiert und angeordnet ist, dass sie eine zweite Öffnung in die Katheter aufnehmende Kammer definiert, wenn der zweite Endabschnitt der Verpackung durch die einleitenden Mittel für die zweite Öffnung manuell geöffnet wird, wobei die zweite Öffnung eine Querschnittsgröße aufweist, die kleiner ist als die Querschnittsgröße des Drainageendabschnitts des Katheters, um zu verhindern, dass der Drainageendabschnitt des Katheters dort hindurch geht, während das Drainieren von Urin dort hindurch ermöglicht wird.

14. Dauergeschmierter Harnblasenkatheter und Verpackungssystem gemäß einem der vorhergehenden Ansprüche, wobei die Verpackung ferner eine Grundschicht an Material, die eine Länge und Breite aufweist, die der Länge und Breite der Verpackung entspricht; und eine Deckschicht an Material, die eine Länge und Breite aufweist, die der Länge und Breite der Verpackung entspricht, umfasst, wobei die Grundschicht und die Deckschicht miteinander versiegelt sind, um die Katheter aufnehmende Kammer zwischen der Grundschicht und der Deckschicht zu formen.

15. Dauergeschmierter Harnblasenkatheter und Verpackungssystem gemäß einem der Ansprüche 4, 5, 7 und 9 bis 12, wobei das Führungsmittel einen vorgeformten Führungsstreifen beinhaltet, der an eine Seite der Verpackung, angrenzend an den ersten Endabschnitt gebunden ist, so dass das Führungselement angrenzend an die Katheter aufnehmende Kammer in der Verpackung liegt, bevor die Verpackung geöffnet wird, und sich über die Öffnung in dem ersten Endabschnitt der Verpackung hinaus erstreckt, wenn die Verpackung geöffnet wird, wobei der Führungsstreifen dort hinüber eine Führungsaussparung formt, die innerhalb der Katheter aufnehmenden Kammer befindlich ist und im Allgemeinen auf die erste Achse der Verpackung ausgerichtet ist, wobei die Führungsaussparung eine Querschnittsgröße und -form aufweist, um den Katheter dort hindurch gleitbar aufzunehmen, so dass eine Katheter aufnehmende Öffnung zwischen der Führungsaussparung und der Seite der Verpackung gegenüber des Führungsstreifens definiert ist, wodurch der Führungsstreifen und die Seite der Verpackung gegenüber dem Führungsstreifen manuell aufeinander zu gedrückt werden können, um zu bewirken, dass der Katheter innerhalb der Verpackung ergriffen werden kann und der Katheter losgelassen wird, um frei durch die Katheter aufnehmende Öffnung zu gleiten, wenn der Führungsstreifen und die gegenüberliegende Seite der Verpackung losgelassen werden, um das Passieren des Katheters dort hindurch zu ermöglichen.

16. Dauergeschmierter Harnblasenkatheter und Verpackungssystem gemäß Anspruch 5, wobei die den Katheter aufnehmende Kammer eine Querschnittsgröße und -form entlang ihrer Länge aufweist, so dass der größte Querschnitt des Katheters dort entlang verläuft, damit die Verpackung entlang dem Katheter gezogen werden kann, bis der erste Endabschnitt der Katheter aufnehmenden Kammer angrenzend an den Drainageendeabschnitt des Katheters befindlich ist, wobei der Drainageendabschnitt des Katheters noch innerhalb der Katheter aufnehmenden Kammer befindlich ist, wodurch die Verpackung als drainierende Verlängerung des Katheters funktioniert.

17. Ein Verfahren zur Herstellung des dauergeschmierten Harnblasenkatheters und Verpackungssystems gemäß einem der vorhergehenden Ansprüche, das folgende Schritte beinhaltet:
a) Formen einer primären Aussparung in einer Grundschicht an Material, die bemessen ist, um den Katheter darin aufzunehmen, wo die primäre Aussparung ein erstes Ende und eine kleinere sekundäre Führungsaussparung innerhalb der primären Aussparung angrenzend an das erste Ende der ersten Aussparung, das bemessen ist, um den vorspringenden Endabschnitt des Katheters dort hindurch gleitbar aufzunehmen, aufweist;
b) Setzen des Katheters in die primäre und sekundäre Aussparung, so dass der vorspringende Endabschnitt des Katheters an dem ersten Ende der primären Aussparung befindlich ist und innerhalb der sekundären Aussparung liegt;
c) Hinterlegen eines Schmiermittels in der primären und sekundären Aussparung, so dass sich das Schmiermittel von einem ersten Ende der primären und sekundären Aussparung um einen vorgegebenen Abstand zu dem entgegengesetzten Ende der primären und sekundären Aussparung hin erstreckt, so dass das Schmiermittel die primäre und sekundäre Aussparung nicht überfüllt, wenn der Katheter in der primären und sekundären Aussparung befindlich ist;
d) Legen einer Deckschicht an Material über die Grundschicht an Material, so dass die Deckschicht die primäre und sekundäre Aussparung abdeckt; und
e) Versiegeln der Deckschicht mit der Grundschicht um die primäre und sekundäre Aussparung herum, um eine abgedichtete Katheter aufnehmende Kammer, in der sich der Katheter und das Schmiermittel befinden, zu formen.

18. Ein Verfahren zur Verwendung des dauergeschmierten Harnblasenkatheters und Verpackungssystems gemäß einem der Ansprüche 1 bis 16, das folgende Schritte beinhaltet:
a) Anfertigen einer ersten Öffnung durch den ersten Endabschnitt der Verpackung, die ausreicht, damit der vorspringende Endabschnitt des Katheters dort hindurch verlaufen kann;
b) Anfertigen einer zweiten Öffnung durch die Verpackung in der Nähe der Ablassöffnung des Katheters; und
c) nachdem die erste und zweite Öffnung angefertigt worden sind, manuelles Ausfahren des vorspringenden Endabschnitts des Katheters aus der Verpackung durch das manuelle Ergreifen und Loslassen des Katheters in dem Harnblasenkatheter-Führungsdurchlass durch die Verpackung und das Bedienen des Katheters durch die Verpackung.

19. Verfahren gemäß Anspruch 18, wobei der Katheter nicht gänzlich aus der Verpackung entfernt wird.

20. Verfahren gemäß Anspruch 18 oder 19, wobei der vorspringende Endabschnitt des Katheters in einem geraden Zustand bereitgestellt ist.

## Revendications

1. Un assemblage de sonde urinaire prélubrifiée et d'emballage destiné à être utilisé pour drainer la vessie par l'urètre, la sonde urinaire prélubrifiée étant extensible depuis l'emballage, et l'emballage comportant une première et une deuxième portion d'extrémité, dans lequel l'assemblage de sonde urinaire prélubrifiée et d'emballage comporte :
a) un emballage flexible définissant une chambre de réception de sonde dans celui-ci ;
b) une sonde reçue dans la chambre de réception de sonde, la sonde ayant une portion d'extrémité de drainage et une portion d'extrémité en saillie destinée à s'étendre de la chambre de réception de sonde dans l'emballage ;
c) une quantité prescrite de lubrifiant ; et
d) un moyen de lubrification destiné à amener le lubrifiant à être appliqué sur la portion d'extrémité en saillie de la sonde pour lubrifier la sonde pour l'utilisation,
**caractérisé par le fait que** l'assemblage de sonde urinaire prélubrifée et d'emballage comporte un moyen de contrôle d'extension permettant à la sonde de s'étendre depuis l'emballage, le moyen de contrôle d'extension étant substantiellement capable d'empêcher la sonde de rentrer dans l'emballage par rétraction.

2. L'assemblage de sonde urinaire prélubrifiée et d'emballage de la revendication 1 dans lequel le moyen de contrôle d'extension comprend un moyen de prise permettant à la sonde de s'étendre depuis l'emballage, dans lequel le moyen de prise peut être opéré pour empêcher substantiellement la sonde de rentrer dans l'emballage par rétraction.

3. L'assemblage de sonde urinaire prélubrifiée et d'emballage soit de la revendication 1, soit de la revendication 2 dans lequel le moyen de lubrification est construit et agencé de manière à amener par force le lubrifiant sur la sonde à mesure que la sonde est étendue hors de l'emballage.

4. L'assemblage de sonde urinaire prélubrifiée et d'emballage de n'importe laquelle des revendications 1 à 3 comportant de plus un moyen formant guide comprenant un élément guide préformé définissant un passage guide de sonde urinaire à travers celui-ci, l'emballage définissant un premier axe s'étendant longitudinalement à travers celui-ci.

5. L'assemblage de sonde urinaire prélubrifiée et d'emballage de la revendication 4, dans lequel :
i) l'emballage a la chambre de réception de sonde définie dans celui-ci sous une forme scellée allongée, s'étendant le long du premier axe depuis la première portion d'extrémité jusqu'à la deuxième portion d'extrémité et ayant une longueur prescrite minimale;
ii) la sonde ayant des extrémités opposées et une longueur maximale inférieure à la longueur prescrite minimale de la chambre de réception de sonde dans l'emballage, la sonde définissant un passage de drainage s'étendant de la portion d'extrémité en saillie à la portion d'extrémité de drainage, le passage de drainage définissant une ouverture d'admission pour celle-ci à la portion d'extrémité en saillie et une ouverture de décharge depuis celle-ci à la portion d'extrémité de drainage de sorte que des fluides dans la vessie puissent être drainés par celle-ci lorsque la portion d'extrémité en saillie de la sonde est insérée dans la vessie par l'urètre ; la sonde étant portée dans la chambre de réception de sonde dans un état généralement rectiligne, la portion d'extrémité en saillie de la sonde étant située à une première portion d'extrémité de la chambre de réception de sonde de sorte que la portion d'extrémité en saillie de la sonde puisse sortir de la chambre de réception de sonde lorsque la première portion d'extrémité de la chambre de réception de sonde est ouverte manuellement ; dans lequel le moyen de contrôle d'extension empêche la rétraction de la sonde jusque dans la chambre de réception de sonde ; et
iii) le moyen formant guide étant associé de façon opérationnelle à la première portion d'extrémité de l'emballage et définissant un passage guide de sonde urinaire au sein de la chambre de réception de sonde dont la taille et la forme épousent généralement la portion d'extrémité en saillie de la sonde et étant orienté généralement le long du premier axe à travers l'emballage, le passage guide de sonde urinaire communiquant avec la chambre de réception de sonde et étant associé de façon opérationnelle à la première portion d'extrémité de l'emballage de sorte que le passage guide de sonde urinaire reste scellé jusqu'à ce que la première portion d'extrémité de l'emballage soit ouverte manuellement, après quoi la portion d'extrémité en saillie de la sonde peut sortir de la chambre de réception de sonde de façon à pouvoir coulisser par le passage guide de sonde urinaire pour guider la sonde hors de la chambre de réception de sonde.

6. L'assemblage de sonde urinaire prélubrifiée et d'emballage de n'importe quelle revendication précédente dans lequel l'emballage comprend de plus :
a) un premier moyen de déclenchement d'ouverture à la première portion d'extrémité de l'emballage destiné à contrôler l'ouverture manuelle de la chambre de réception de sonde à la première portion d'extrémité de l'emballage, et
b) un deuxième moyen de déclenchement d'ouverture à la deuxième portion d'extrémité de l'emballage destiné à contrôler l'ouverture manuelle de la chambre de réception de sonde à la deuxième portion d'extrémité de l'emballage.

7. L'assemblage de sonde urinaire prélubrifiée et d'emballage de soit la revendication 4, soit la revendication 5 dans lequel le moyen formant guide est situé au sein de la chambre de réception de sonde dans l'emballage de sorte que la portion d'extrémité en saillie de la sonde soit maintenue au sein du passage guide de sonde urinaire lorsque la chambre de réception de sonde est scellée.

8. L'assemblage de sonde urinaire prélubrifiée et d'emballage de n'importe quelle revendication précédente comportant de plus un agencement de prise construit et agencé de sorte que la sonde puisse être agrippée manuellement de façon sélective au sein de l'emballage pour manipuler la sonde et étendre de façon sélective la sonde depuis l'emballage.

9. L'assemblage de sonde urinaire prélubrifiée et d'emballage de n'importe laquelle des revendications 4, 5 et 7 dans lequel le moyen de contrôle d'extension permet à la sonde de traverser le passage guide de sonde urinaire dans un premier sens de sorte que la portion d'extrémité en saillie de la sonde puisse être poussée manuellement de façon sélective pour sortir de l'emballage par le passage guide de sonde urinaire, le moyen de contrôle d'extension retenant de plus la sonde pour qu'elle ne traverse pas le passage guide de sonde urinaire dans un deuxième sens opposé au premier sens pour empêcher substantiellement de faire rentrer par inadvertance la portion d'extrémité en saillie de la sonde par rétraction dans la chambre de réception de sonde dans l'emballage alors que la sonde est manipulée manuellement dans l'emballage pour étendre de façon sélective la sonde depuis l'emballage.

10. L'assemblage de sonde urinaire prélubrifiée et d'emballage de n'importe laquelle des revendications 4, 5, 7 et 9 dans lequel le moyen de contrôle d'extension contrôle la traversée par la sonde du passage guide de sonde urinaire pour permettre à la portion d'extrémité en saillie de la sonde d'être poussée manuellement de façon sélective pour sortir de l'emballage par le passage guide de sonde urinaire, le moyen de contrôle d'extension comprenant un moyen de retenue destiné à empêcher substantiellement la traversée par la sonde du passage guide de sonde urinaire jusqu'à ce qu'une force axiale supérieure à un niveau de seuil minimal prescrit soit appliquée sur la sonde, ce qui tend à faire traverser à la sonde le passage guide de sonde urinaire.

11. L'assemblage de sonde urinaire prélubrifiée et d'emballage de la revendication 10 dans lequel le niveau de seuil minimal prescrit est sélectionné pour être suffisant pour permettre à l'emballage flexible d'être déplacé par rapport à la sonde dans la chambre de réception de sonde pour permettre à la sonde d'être manipulée manuellement au sein de la chambre de réception de sonde de manière à étendre de façon sélective la sonde depuis l'emballage.

12. L'assemblage de sonde urinaire prélubrifiée et d'emballage de la revendication 9 dans lequel le moyen de contrôle d'extension comporte un moyen formant embrayage unidirectionnel destiné à permettre à la sonde de se déplacer dans le premier sens, tout en empêchant substantiellement le déplacement de la sonde dans le deuxième sens.

13. L'assemblage de sonde urinaire prélubrifiée et d'emballage de la revendication 6 dans lequel l'emballage est de plus construit et agencé de manière à définir une deuxième ouverture dans la chambre de réception de sonde lorsque la deuxième portion d'extrémité de l'emballage est ouverte manuellement par l'intermédiaire du deuxième moyen de déclenchement d'ouverture, la deuxième ouverture ayant une taille en coupe transversale inférieure à la taille en coupe transversale de la portion d'extrémité de drainage de la sonde pour empêcher la portion d'extrémité de drainage de la sonde de passer par celle-ci, tout en permettant à l'urine d'être drainée au travers de celle-ci.

14. L'assemblage de sonde urinaire prélubrifiée et d'emballage de n'importe quelle revendication précédente dans lequel l'emballage comprend de plus une feuille de base en matériau dont une longueur et une largeur correspondent à la longueur et la largeur de l'emballage; et une feuille de couverture en matériau dont une longueur et une largeur correspondent à la longueur et la largeur de l'emballage, la feuille de base et la feuille de couverture étant scellées l'une à l'autre pour former la chambre de réception de sonde entre la feuille de base et la feuille de couverture.

15. L'assemblage de sonde urinaire prélubrifiée et d'emballage de n'importe laquelle des revendications 4, 5, 7 et 9 à 12 dans lequel le moyen formant guide comporte une bande guide préformée collée à un côté de l'emballage adjacent à la première portion d'extrémité de telle sorte que l'élément guide se trouve adjacent à la chambre de réception de sonde dans l'emballage avant que l'emballage ne soit ouvert et s'étende de part et d'autre de l'ouverture dans la première portion d'extrémité de l'emballage lorsque l'emballage est ouvert, la bande guide formant un renfoncement guide de part et d'autre de celle-cl situé au sein de la chambre de réception de sonde et généralement en alignement sur le premier axe de l'emballage, le renfoncement guide ayant une taille et une forme en coupe transversale pour recevoir la sonde à travers celui-ci de façon à ce qu'elle puisse coulisser de telle sorte qu'une ouverture de réception de sonde soit définie entre le renfoncement guide et le côté de l'emballage opposé à la bande guide, grâce à quoi la bande guide et le côté de l'emballage qui est opposé à la bande guide peuvent être comprimés manuellement l'un vers l'autre pour amener la sonde à être saisie au sein de l'emballage et la sonde sera libérée de manière à coullsser librement par l'ouverture de réception de sonde lorsque la bande guide et le côté opposé de l'emballage sont libérés pour permettre à la sonde de passer au travers de celle-ci.

16. L'assemblage de sonde urinaire prélubrifiée et d'emballage de la revendication 5 dans lequel la chambre de réception de sonde a une taille et une forme en coupe transversale sur sa longueur de telle sorte que la plus grande coupe transversale de la sonde passera le long de celle-ci de telle sorte que l'emballage puisse être tiré le long de la sonde jusqu'à ce que la première portion d'extrémité de la chambre de réception de sonde soit située adjacente à la portion d'extrémité de drainage de la sonde, la portion d'extrémité de drainage de la sonde étant toujours située au sein de la chambre de réception de sonde, grâce à quoi l'emballage fait office d'extension de drainage pour la sonde.

17. Un procédé de production de l'assemblage de sonde urinaire prélubrifiée et d'emballage de n'importe quelle revendication précédente comportant les étapes de :
a) former un renfoncement primaire dans une feuille de base en matériau dimensionnée pour recevoir la sonde dans celui-ci où le renfoncement primaire a une première extrémité et un renfoncement guide secondaire plus petit au sein du renfoncement primaire adjacent à la première extrémité du renfoncement primaire dimensionné pour recevoir la portion d'extrémité en saillie de la sonde à travers celui-ci de façon à ce qu'elle puisse coulisser ;
b) placer la sonde dans les renfoncements primaire et secondaire de sorte que la portion d'extrémité en saillie de la sonde soit située à la première extrémité du renfoncement primaire et se trouve au sein du renfoncement secondaire ;
c) déposer un lubrifiant dans les renfoncements primaire et secondaire de sorte que le lubrifiant s'étende d'une première extrémité des renfoncements primaire et secondaire sur une distance prescrite vers l'extrémité opposée des renfoncements primaire et secondaire et de sorte que le lubrifiant ne déborde pas des renfoncements primaire et secondaire lorsque la sonde est située dans les renfoncements primaire et secondaire;
d) placer une feuille de couverture en matériau par-dessus la feuille de base en matériau de sorte que la feuille de couverture recouvre les renfoncements primaire et secondaire ; et,
e) sceller la feuille de couverture à la feuille de base autour des renfoncements primaire et secondaire de manière à former une chambre de réception de sonde scellée, la sonde et le lubrifiant étant dans celle-ci.

18. Un procédé d'utilisation de l'assemblage de sonde urinaire prélubrifiée et d'emballage de n'importe laquelle des revendications 1 à 16 comportant les étapes de :
a) pratiquer une première ouverture à travers la première portion d'extrémité de l'emballage suffisante pour que la portion d'extrémité en saillie de la sonde passe au travers de celle-ci ;
b) pratiquer une deuxième ouverture à travers l'emballage au voisinage de l'ouverture de décharge de la sonde ; et
c) une fois les première et deuxième ouvertures pratiquées, étendre manuellement la portion d'extrémité en saillie de la sonde pour qu'elle sorte de l'emballage en agrippant et libérant manuellement la sonde dans le passage guide de sonde urinaire à travers l'emballage et manipulant la sonde à travers l'emballage.

19. Le procédé de la revendication 18 dans lequel la sonde n'est pas entièrement enlevée de l'emballage.

20. Le procédé de l'une ou l'autre des revendications 18 et 19 dans lequel la portion d'extrémité en saillie de la sonde est fournie dans un état rectiligne.
